# EUROPÄISCHE PATENTSCHRIFT

(11) **EP 2 401 020 B1**
(45) Veröffentlichungstag und Bekanntmachung des Hinweises auf die Patenterteilung: **16.12.2020**
(21) Anmeldenummer: 10704082.6
(22) Anmeldetag: 17.02.2010
(51) Int. Cl.: A61M 25/10, A61M 25/00

(54) **KATHETER**
CATHETER
CATHÉTER

(30) Priorität: 27.02.2009 CH 2952009
(43) Veröffentlichungstag der Anmeldung: 04.01.2012
(73) Patentinhaber: SIS Medical AG, 8500 Frauenfeld (CH)
(72) Erfinder: SCHWAGER, Michael, CH-8404 Winterthur (CH); VOGEL, Rolf, CH-3067 Boll-Sinneringen (CH)
(74) Vertreter: Keller Schneider Patent- und Markenanwälte AG (Bern)
(86) Internationale Anmeldenummer: PCT/CH2010/000041
(87) Internationale Veröffentlichungsnummer: WO 2010/096940

(56) Entgegenhaltungen:
- WO-A-00/69499
- WO-A-93/05841

## Beschreibung

### Technisches Gebiet

Die Erfindung betrifft einen Katheter zur Einführung in ein menschliches und/oder tierisches Hohlorgan, umfassend einen Katheterschaft mit einem Lumen, welches in eine vordere Öffnung an einem distalen Ende des Katheterschafts mündet, wobei der Katheterschaft wenigstens zwei vom distalen Ende beabstandete und in das Lumen mündende Seitenöffnungen aufweist.

### Stand der Technik

Katheter sind üblicherweise Röhrchen und/oder Schläuche verschiedenen Durchmessers, zum Beispiel aus Kunststoff, Latex, Silikon, Metall oder Glas. Mit ihnen können Hohlorgane, wie zum Beispiel Harnröhre, Speiseröhre, Magen, Darm, Gallengänge, Gefässe und/oder blutführende Adern, wie z. B. Herzarterien, von Mensch und/oder Tier, sondiert, durchstossen, entleert, gefüllt und/oder gespült werden. Dies geschieht aus insbesondere diagnostischen und/oder therapeutischen Gründen.

Mit Hilfe von Kathetern können insbesondere auch interventionelle und/oder diagnostische Elemente, insbesondere in Form von drahtförmigen Elementen, z. B. spezielle Sondendrähte für diagnostische Zwecke, in Hohlorgane eingeführt werden. Der Katheter dient dabei als Führung für ein beispielsweise aufgrund seiner mechanischen Eigenschaften nicht direkt in das Hohlorgan einbringbares drahtförmiges Element.

Hierfür werden in der Praxis oft sogenannte Monorail-Katheter verwendet, welche auf dem Schnellaustausch-System (auch "Rapid-exchange-System" genannt) beruhen. Derartige Katheter werden einem vorgängig in das Hohlorgan eingeführten Führungsdraht folgend in das Hohlorgan eingebracht. Im Unterschied zu Kathetern bei welchen der Führungsdraht zentral durch den gesamten Katheter hindurch geführt wird (nach dem klassischen "Overthe-Wire-System"), verläuft der Führungsdraht bei einem Monorail-Katheter lediglich in einem distalen Bereich zwischen der Katheterspitze und einer von der Katheterspitze beabstandeten seitlichen Öffnung durch den Katheter hindurch. Als besonderer Vorteil kann ein Monorail-Katheter aus dem Hohlorgan zurückgezogen (und gegebenenfalls wieder in dieses hineingeschoben) werden, wobei der Führungsdraht im Hohlorgan liegen bleiben kann, ohne dass ein Verlängerungsdraht am Führungsdraht angesetzt werden muss. Damit wird die Handhabung stark vereinfacht.

Ist der Monorail-Katheter mit seinem distalen Ende bzw. der Katheterspitze an der gewünschten Stelle im Hohlorgan platziert, kann der Führungsdraht zurückgezogen werden, so dass er aus der seitlichen Öffnung aus dem Katheter austritt, und das drahtförmige Element kann vom proximalen Ende her durch ein zentrales Lumen des Katheters hindurch in das Hohlorgan eingeführt werden. Anschliessend kann die entsprechende diagnostische und/oder therapeutische Behandlung vorgenommen werden.

Grundsätzlich wäre es für verschiedene Anwendungen vorteilhaft, den Monorail-Katheter nach erfolgter Positionierung des drahtförmigen Elements vollständig aus dem Hohlorgan zurückzuziehen, so dass lediglich das drahtförmige Element im Hohlorgan zurück bleibt. Wird der Monorail-Katheter z. B. durch einen fix gelegten Herzkranzkatheter hindurch in das Hohlorgan eingeführt, reduziert der Monorail-Katheter nämlich den freien Querschnitt des Herzkranzkatheters massiv, was beispielsweise das Einleiten von Fluiden durch den Herzkranzkatheter stark erschwert. Insbesondere die Einleitung von hochviskosen Kontrastmitteln, welche beispielsweise bei der intravaskulären Bildgebung (z. B. bei der optischen Kohärenztomographie) in das Hohlorgan eingebracht werden müssen, wird durch den vorliegenden Monorail-Katheter stark erschwert.

Prinzipiell besteht natürlich die Möglichkeit, den Sondendraht entsprechend länger auszubilden, so dass der Monorail-Katheter vollständig zurück gezogen werden kann. Dies ist aber aus wirtschaftlichen Gründen nachteilig und zudem komplizieren die Überlängen der drahtförmigen Elemente die Handhabung in der Praxis. Das Anbringen von Verlängerungsstücken, wie dies bei den Führungsdrähten gemacht wird, ist bei Sondendrähten meist nicht möglich. Sondendrähte verfügen nämlich am proximalen Ende üblicherweise über ein voluminöses Anschlussstück, welches ein Zurückziehen des Katheters verunmöglicht.

Die WO 00/69499 beschreibt einen Gallen-Katheter mit einer Seitenöffnung, wobei die Seitenöffnung eine flexiblere Handhabung ermöglicht. Austauschbar ist hierbei der Katheter an sich. Eine Kathetervariante mit zwei Seitenöffnungen beschreibt die WO 93/05841. Auch hier kann der Katheter gegen einen anderen ausgetauscht werden. Ein Austausch drahtförmiger Elemente wird erwähnt, er ist jedoch vom Ablauf her nicht verbessert.

Es besteht daher nach wie vor Bedarf nach einem verbesserten Katheter, welcher insbesondere die Einführung von interventionellen und/oder diagnostischen Elementen, insbesondere in Form von drahtförmigen Elementen, in menschliche und/oder tierische Hohlorgane vereinfacht.

### Darstellung der Erfindung

Aufgabe der Erfindung ist es, ein dem eingangs genannten technischen Gebiet zugehörenden Katheter zu schaffen, welcher flexibler einsetzbar ist und insbesondere rationellere Verfahrensabläufe bei der Verwendung von drahtförmigen Elementen in menschlichen und/oder tierischen Hohlorganen zu diagnostischen, therapeutischen und/oder chirurgischen Zwecken ermöglicht.

Die Lösung der Aufgabe ist durch die Merkmale des Anspruchs 1 definiert. Gemäss der Erfindung liegt an einer Aussenseite des Katheterschafts mit einem Lumen in einem Bereich der ersten Seitenöffnung wenigstens eine erste Einführvorrichtung für ein von aussen in die erste Seitenöffnung einzuführendes drahtförmiges Element vor. Zudem weist der Katheter eine zweite Seitenöffnung auf.

Der Katheterschaft ist bevorzugt als flexibles Röhrchen und/oder als Schlauch ausgebildet. Mit Vorteil weist der Katheterschaft einen im Wesentlichen kreisrunden Querschnitt auf. Derartige Katheterschäfte weisen eine besonders gute Einführbarkeit in Hohlorgane auf. Prinzipiell sind aber auch andere Querschnitte, z. B. ovale oder polygonale Querschnitte, denkbar.

Unter dem distalen Ende des Katheterschafts wird dabei insbesondere das in das Hohlorgan einzuführende Ende des Katheterschafts verstanden. Mit anderen Worten wird der Katheter mit dem distalen Ende voran in das Hohlorgan eingeschoben. Entsprechend ist das proximale Ende des Katheterschafts insbesondere das dem Hohlorgan abgewandte Ende des Katheterschafts. Das proximale Ende des Katheterschafts wird somit üblicherweise nicht in das Hohlorgan eingeführt.

Der Begriff Lumen steht in diesem Zusammenhang insbesondere für einen inneren Hohlraum des Katheterschafts, welcher im Besonderen vom proximalen Ende bis zum distalen Ende vollständig durch den Katheterschaft hindurch verläuft. Insbesondere liegt das Lumen als durchgängiger im Wesentlichen zylindrischer Hohlraum vor, wobei dieser jedoch abschnittsweise auch nicht-zylindrisch, verjüngt und/oder aufgeweitet ausgebildet sein kann. Grundsätzlich ist es aber auch möglich, ein Lumen vorzusehen, welches sich nicht durch den gesamten Katheterschaft hindurch erstreckt und z. B. in einem Bereich vor dem proximalen Ende aus dem Katheterschaft austritt.

Ein Material des Katheterschafts besteht wenigstens in einem distalen Abschnitt mit Vorteil aus einem Kunststoff. Kunststoffe sind einerseits ausreichend flexibel und können zudem in vielen Fällen relativ einfach bearbeitet werden. Mit Vorteil ist der Katheterschaft vollständig aus einem Kunststoff gebildet. Insbesondere ein proximaler Abschnitt des Katheterschafts kann jedoch auch aus einem metallischen und/oder einem anderen Material bestehen. Es ist aber auch möglich, für den Katheterschaft anstelle von oder zusätzlich zum Kunststoff andere Materialien zu verwenden.

Unter einem drahtförmigen Element wird in diesem Zusammenhang insbesondere ein biegbarer Körper verstanden, dessen Länge um ein Vielfaches, bevorzugt wenigstens um einen Faktor 100, grösser ist als sein Durchmesser und/oder seine Breite. Das drahtförmige Element kann insbesondere einen kreisrunden Querschnitt aufweisen und muss nicht metallisch sein. Insbesondere ist das drahtförmige Element ein Spezialdraht und/oder Sondendraht für ein diagnostisches Verfahren, und beinhaltet z. B. eine Faseroptik. Mit Vorteil ist das drahtförmige Element ein Sondendraht für eine optische Kohärenztomographie. An einem distalen Ende des drahtförmigen Elements ist dabei insbesondere ein Messkopf angeordnet. Das drahtförmige Element kann aber beispielsweise auch ein herkömmlicher Führungsdraht, insbesondere aus Metall sein.

Der Katheter ist als ein Einlumenkatheter ausgebildet. Derartige Katheter sind insbesondere relativ einfach herstellbar und können mit geringen Abmessungen gefertigt werden. Dadurch lässt sich der Katheter flexibler einsetzen.

Die an der Aussenseite des Katheterschafts vorliegende erste Einführvorrichtung ermöglicht es, ein drahtförmiges Element in einfacher Weise von aussen in die erste Seitenöffnung und damit in das Lumen des Katheters einzuführen. Die erste Einführvorrichtung ist dabei insbesondere derart ausgebildet, dass das drahtförmige Element bei einer Bewegung entlang der ersten Einführvorrichtung auf der Aussenseite des Katheterschafts in die erste Seitenöffnung gelenkt wird. Ein Übergang zwischen der ersten Einführvorrichtung und der ersten Seitenöffnung ist dabei insbesondere naht- und/oder stufenlos ausgebildet.

Der Katheterschaft weist eine in das Lumen mündende zweite Seitenöffnung auf. Damit lassen sich die Verfahrensabläufe bei der Einführung von drahtförmigen Elementen in menschliche und/oder tierische Hohlorgane zu diagnostischen, therapeutischen und/oder chirurgischen Zwecken weiter rationalisieren und die erfindungsgemässen Katheter werden dadurch noch flexibler einsetzbar. Es ist denkbar, mehr als zwei Seitenöffnungen vorzusehen. Dabei können noch komplexere Verfahrensabläufe mit mehreren gleichzeitig im Katheter vorliegenden drahtförmigen Elementen realisiert werden. Die weiteren Seitenöffnungen weisen mit Vorteil eine entsprechende Einführvorrichtung auf.

Aufgrund der Einführvorrichtung können auch sehr dünne drahtförmige Elemente auf Anhieb von aussen in Seitenöffnungen mit geringen Durchmessern eingeführt werden. Seitenöffnungen von herkömmlichen Kathetern sind üblicherweise dafür ausgelegt, einen vom distalen Ende her eingeführten Führungsdraht aus dem Lumen nach aussen zu führen. Wollte man bei einem derartigen Katheter ein drahtfrömiges Element von aussen in eine Seitenöffnung einbringen, wäre dies nur in einem langwierigen Einfädelprozess und unter Verwendung von Vergrösserungslinsen oder dergleichen realisierbar.

Durch die erfindungsgemässe Lösung können die Durchmesser der Seitenöffnungen minimal gehalten werden, und dennoch wird eine gute Einführbarkeit für drahtförmige Elemente in die Seitenöffnungen erreicht. Möglichst kleine Seitenöffnungen kommen unter anderem der Stabilität des Katheterschafts und der Einführbarkeit des Katheters in das Hohlorgan insgesamt zu Gute.

Die erfindungsgemässe Lösung schafft eine zusätzliche und praktische Möglichkeit, ein drahtförmiges Element in das Lumen des Katheters einzubringen. Drahtförmige Elemente lassen sich somit nicht nur wie bekannt vom proximalen Ende des Katheterschafts her in das Lumen einführen, sondern ebenso praktikabel auch durch eine der Seitenöffnungen im Bereich des distalen Endes. Damit lassen sich bei der Einführung von drahtförmigen Elementen in menschliche und/oder tierische Hohlorgane zu diagnostischen, therapeutischen und/oder chirurgischen Zwecken insbesondere neue und rationellere Verfahrensabläufe realisieren. Ebenso sind die erfindungsgemässen Katheter insgesamt flexibler einsetzbar.

Die zweite Seitenöffnung ist in einer proximalen Richtung von der ersten Seitenöffnung beabstandet. Insbesondere bevorzugt ist die zweite Seitenöffnung dabei diametral gegenüberliegend zur ersten Seitenöffnung angeordnet. Vorteilhaft liegt an der Aussenseite des Katheterschafts zudem eine zweite Einführvorrichtung für ein von aussen in die zweite Seitenöffnung einzuführendes zweites drahtförmiges Element vor.

Sind die beiden Seitenöffnungen diametral gegenüberliegend angeordnet, wird die Führung von mehreren drahtförmigen Elementen vereinfacht. Die beiden drahtförmigen Elemente können in diesem Fall nämlich nach dem Durchgang durch die jeweiligen Seitenöffnungen an gegenüberliegenden Begrenzungsflächen durch das Lumen geführt werden. Ein Überkreuzen der drahtförmigen Elemente wird so vermieden.

Prinzipiell ist es auch denkbar, auf diametral gegenüberliegende Seitenöffnungen zu verzichten und Seitenöffnungen auf einer gemeinsamen Seite anzuordnen. Dies kann unter Umständen aber die Führung von drahtförmigen Elementen im Lumen erschweren. Liegen z. B. drei oder noch mehr Seitenöffnungen vor, kann es auch vorteilhaft sein, diese gleichmässig um die Aussenseite herum zu verteilen.

Ebenso ist es denkbar, mehr als zwei Seitenöffnungen vorzusehen. Dabei können noch komplexere Verfahrensabläufe mit mehreren gleichzeitig im Katheter vorliegenden drahtförmigen Elementen realisiert werden.

Die erste Einführvorrichtung liegt mit Vorteil in einem dem proximalen Ende zugewandten und an die erste Seitenöffnung angrenzenden Bereich der Aussenseite des Katheterschafts vor. Damit kann das drahtförmige Element aus einem proximalen Bereich in Richtung des distalen Endes in die erste Seitenöffnung eingeführt werden. Da ein durch eine Seitenöffnung in das Lumen eingeführtes drahtförmiges Element üblicherweise ebenfalls in Richtung zum distalen Ende geschoben werden müssen, erleichtert eine derartige Anordnung der ersten Einführvorrichtung die Einführung des drahtförmigen Elements insgesamt, da die Bewegungsrichtung des drahtförmigen Elements in der ersten Einführvorrichtung im Lumen im Wesentlichen gleichgerichtet sind.

Grundsätzlich ist es aber auch denkbar, die erste Einführvorrichtung beispielsweise in einem dem distalen Ende zugewandten und an die erste Seitenöffnung angrenzenden Bereich der Aussenseite des Katheterschafts vorzusehen. Ebenso kann die erste Einführvorrichtung prinzipiell in einem Bereich seitlich der ersten Seitenöffnung vorliegen. Unter Umständen muss das drahtförmige Element jedoch nach der Einführung in die erste Seitenöffnung in eine andere Richtung weiter geschoben werden.

Mit Vorteil ist die zweite Einführvorrichtung der zweiten Seitenöffnung im Wesentlichen baugleich ausgebildet wie die vorstehend beschriebene erste Einführvorrichtung der ersten Seitenöffnung. Damit kann auch in die zweite Seitenöffnung ein drahtförmiges Element von aussen in das Lumen eingeführt werden. Die bei der ersten Einführvorrichtung spezifischen Ausgestaltungen lassen sich entsprechend auch bei der zweiten Einführvorrichtung verwirklichen.

Es ist aber auch denkbar, die zweite Einführvorrichtung anders auszubilden als die erste Einführvorrichtung. Die bei der ersten Einführvorrichtung genannten Alternativen und/oder spezifischen Ausführungen können auch bei der zweiten Einführvorrichtung realisiert werden. Damit kann gegebenenfalls die Herstellung des Katheters vereinfacht werden.

Bevorzugt ist die erste Einführvorrichtung als eine sich zur ersten Seitenöffnung hin erstreckende Führungsrinne ausgebildet. Die Führungsrinne verläuft dabei insbesondere parallel zu einer Längsmittelachse des Lumens und besonders bevorzugt entspricht eine Breite der Führungsrinne im Wesentlichen einem Durchmesser der ersten Seitenöffnung. Die Führunsgrinne liegt insbesondere als konkave Einbuchtung im Katheterschaft vor. Eine Führungsrinne stellt eine relativ einfache aber dennoch äusserst zweckmässige und kompakte Führungsvorrichtung dar.

Eine Führungsrinne wird z. B. durch zwei beabstandete Führungselemente gebildet, welche insbesondere parallel zueinander ausgerichtet sind. Wird ein drahtförmiges Element mit seinem distalen Ende in die Führungsrinne bzw. in den Zwischenraum zwischen den beiden Führungselementen geschoben und zur Seitenöffnung hin bewegt, wird das drahtförmige Element, insbesondere aufgrund der beiden Führungselemente zur Seitenöffnung geführt. Entspricht die Breite der Führungsrinne im Wesentlichen dem Durchmesser der Seitenöffnung, wird das drahtförmige Element dabei automatisch in die Seitenöffnung hinein gelenkt.

Es ist zudem auch möglich eine Führungsrinne vorzusehen, welche die erste Seitenöffnung in proximaler als auch in distaler Richtung überragt. In diesem Fall kann ein drahtförmiges Element wahlweise aus proximaler als auch aus distaler Richtung in die Seitenöffnung eingeführt werden.

Auch vorteilhaft kann es sein, eine Führungsrinne mit zwei schräg zueinander stehenden Führungselementen vorzusehen, wobei sich ein Abstand zwischen den beiden Führungselementen beispielsweise zur Seitenöffnung hin stetig vermindert. Dadurch wird ein konisch zulaufender Führungsbereich zwischen den beiden Führungselementen geschaffen, der unter Umständen das Einschieben des drahtförmigen Elements in die Führungsrinne vereinfacht. Ebenso ist es denkbar, die Führungsrinne in einer Richtung schräg zur Längsmittelachse auszubilden. Die Anbringung und/oder Ausformung der Führungsrinne kann dadurch aber erschwert werden.

Grundsätzlich ist es auch denkbar, ein an die Seitenöffnung anschliessenden trichterförmigen Rohrabschnitt als Einführhilfe vorzusehen. Dadurch liegen jedoch unter Umständen zusätzliche und vorstehende Bereiche am Katheterschaft vor, was die Einführbarkeit des Katheters in ein Hohlorgan verschlechtert.

Besonders bevorzugt ist die Führungsrinne als ein konkav gewölbter Bereich der Aussenseite des Katheterschafts ausgebildet, wobei der konkav gewölbte Bereich bevorzugt in Richtung zum distalen Ende eine kontinuierlich zunehmende Tiefe aufweist. Die Führungsrinne geht dabei mit Vorteil nahtlos und/oder stufen- bzw. kantenlos in die Seitenöffnung über.

Konkav gewölbte Bereiche lassen sich relativ einfach in die Aussenseite des Katheterschafts einbringen. So kann z. B. der Katheterschaft erwärmt und mit Hilfe eines auf die Aussenseite gepressten Drahtstücks umgeformt werden. Wird die Führungsrinne als konkav gewölbter Bereich der Aussenseite ausgebildet, können die hervorstehenden Bereiche, z. B. hervorstehende Kanten oder Stufen, am Katheterschaft zudem auf ein Minimum reduziert werden, was der Einführbarkeit in Hohlorgane zu Gute kommt.

Weist die Führungsrinne eine kontinuierlich zunehmende Tiefe auf, ist es insbesondere möglich, einen stufenlosen Übergang von der Aussenseite in die Führungsrinne zu realisieren. Zudem ergibt sich in einem Bereich unmittelbar vor der Seitenöffnung eine bestmögliche Führung des einzuführenden drahtförmigen Elements, was der Einführbarkeit zu Gute kommt.

Grundsätzlich ist es aber auch denkbar, z. B. zwei beabstandete rippenartige Vorstände als Führungsrinne bzw. Einführvorrichtung an der Aussenseite des Katheterschafts anzubringen. Damit entfallen aber unter Umständen die vorstehend genannten Vorteile der konkav gewölbten Bereiche.

Bevorzugt sind die Seitenöffnungen jeweils zum proximalen Ende hin ausgerichtet. Mit anderen Worten zeigt ein Normalenvektor einer Öffnungsfläche einer Seitenöffnung mit Vorteil vom distalen Ende weg und der Normalenvektor steht bevorzugt schräg zur Längsmittelachse des Lumens und/oder des Katheterschafts.

Eine derartige Anordnung ist insbesondere im Zusammenspiel mit einer Einführvorrichtung, welche in einem dem proximalen Ende zugewandten und an die erste Seitenöffnung angrenzenden Bereich der Aussenseite des Katheterschafts vorliegt, vorteilhaft. Ein drahtförmiges Element welches in diesem Fall durch die Einführvorrichtung geschoben wird, kann so noch einfacher in die Seitenöffnung eingeführt werden, da die Seitenöffnung in die Richtung des durch die Einführvorrichtung bewegten drahtförmigen Elements geneigt ist.

Zudem wird dabei die Verbiegung des einzuführenden drahtförmigen Elements im Bereich der Seitenöffnung reduziert, was wiederum die Reibungskräfte zwischen dem drahtförmigen Element und der Seitenöffnung vermindert.

Prinzipiell können aber eine oder mehrere Seitenöffnungen auch senkrecht zur Längsmittelachse ausgerichtet sein. Allerdings entfallen in diesem Fall die vorstehend genannten Vorteile gegebenenfalls.

In einer weiteren bevorzugten Variante ist in einem Bereich der ersten Seitenöffnung im Lumen ein seitlicher und in die erste Seitenöffnung mündender erster kanalartiger Bereich ausgebildet, so dass insbesondere neben dem ersten kanalartigen Bereich ein erster Durchgangsbereich im Lumen frei bleibt. Mit anderen Worten liegt im Lumen im Bereich der ersten Seitenöffnung damit insbesondere eine erste sogenannte Y-Weiche bzw. eine erste Verzweigung vor. Ein dem distalen Ende zugewandter Abschnitt des ersten kanalartigen Bereichs verläuft zudem mit Vorteil im Wesentlichen parallel zur Längsachse des Lumens.

Ein durch die vordere Öffnung in das Lumen eingeführtes drahtförmiges Element, z. B. ein Führungsdraht, kann beispielsweise durch Biegung des Katheterschafts in den kanalartigen Bereich gelenkt werden, von wo aus er direkt in die Seitenöffnung gelangt. Dadurch kann die Flexibilität des erfindungsgemässen Katheters weiter erhöht werden, da ein drahtförmiges Element in einfacher Art und Weise sowohl aus dem Lumen durch die Seitenöffnung nach aussen als auch von aussen durch die Seitenöffnung in das Lumen hinein führbar ist. Mit Vorteil weist der kanalartige Bereich einen Innendurchmesser auf, welcher im Wesentlichen auf den Aussendurchmesser des durchzuführenden drahtförmigen Elements abgestimmt ist.

Alternativ kann das drahtförmige Element in den ersten Durchgangsbereich neben dem ersten kanalartigen Bereich gelenkt werden, wodurch das drahtförmige Element weiter in Richtung zum proximalen Ende durch das Lumen führbar ist. Mit Vorteil weist der erste Durchgangsbereich einen Innendurchmesser auf, welcher im Wesentlichen auf den Aussendurchmesser des durchzuführenden drahtförmigen Elements abgestimmt ist.

Grundsätzlich kann aber auch auf einen kanalartigen Bereich verzichtet werden, oder es kann eine anders ausgebildete Vorrichtung zum Führen des drahtförmigen Elements im Lumen angeordnet werden. Dies erschwert aber unter Umständen das Durchführen des drahtförmigen Elements aus dem Lumen durch die Seitenöffnung nach aussen.

Bevorzugt ist zudem in einem Bereich der zweiten Seitenöffnung im Lumen ein seitlicher und in die zweite Seitenöffnung mündender zweiter kanalartiger Bereich ausgebildet, so dass insbesondere neben dem zweiten kanalförmigen Bereich ein zweiter Durchgangsbereich des Lumes vorliegt. Dadurch liegt mit anderen Worten im Lumen im Bereich der zweiten Seitenöffnung insbesondere eine zweite Y-Weiche bzw. eine zweite Verzweigung vor. Ein dem distalen Ende zugewandter Abschnitt des zweiten kanalartigen Bereichs verläuft ebenfalls mit Vorteil im Wesentlichen parallel zur Längsachse des Lumens.

Wie beim ersten kanalartigen Bereich kann ein durch die vordere Öffnung in das Lumen eingeschobenes und neben dem ersten kanalartigen Bereich vorbei bzw. durch den ersten Durchgangsbereich durchgeführtes drahtförmiges Element z. B. durch Krümmung des Katheterschafts in den zweiten kanalartigen Bereich eingeführt werden, von wo aus er direkt in die zweite Seitenöffnung gelenkt wird. Dadurch kann die Flexibilität des erfindungsgemässen Katheters weiter erhöht werden, da drahtförmige Elemente in einfacher Art und Weise sowohl aus dem Lumen durch die zweite Seitenöffnung nach aussen als auch von aussen durch die zweite Seitenöffnung in das Lumen hinein führbar sind.

Alternativ kann das drahtförmige Element in den zweiten Durchgangsbereich neben dem zweiten kanalartigen Bereich gelenkt werden, wodurch das drahtförmige Element weiter in Richtung zum proximalen Ende durch das Lumen gelangt. Mit Vorteil weist der zweite Durchgangsbereich einen Innendurchmesser auf, welcher im Wesentlichen auf den Aussendurchmesser des durchzuführenden drahtförmigen Elements bzw. Führungsdraht abgestimmt ist.

Es liegt aber auch im Rahmen der Erfindung, auf einen zweiten kanalartigen Bereich zu verzichten, wobei unter Umständen aber das Durchführen eines drahtförmigen Elements aus dem Lumen durch die zweite Seitenöffnung nach aussen erschwert wird.

Besonders bevorzugt ist ein Innendurchmesser des Lumens in einem Bereich zwischen dem distalen Ende und der ersten Seitenöffnung grösser ausgebildet als in den übrigen Bereichen des Lumens. Besonders bevorzugt ist der Innendurchmesser des Lumens in dem Bereich zwischen dem distalen Ende und der ersten Seitenöffnung dabei derart ausgebildet, dass ein mit dem Katheter einzuführendes drahtförmiges Element, z. B. ein Sondendraht, und ein mit dem Katheter zu verwendender Führungsdraht im Lumen nebeneinander Platz finden. Damit können das drahtförmiges Element und der Führungsdraht gleichzeitig im Bereich zwischen dem distalen Ende und der ersten Seitenöffnung vorliegen und/oder nebeneinander vorbeigeschoben werden. Diese Massnahme erhöht insbesondere die Flexibilität des erfindungsgemässen Katheters und reduziert die Dimensionen des Katheters in den übrigen Bereichen auf ein Minimum.

In einer weiteren vorteilhaften Variante ist der Innendurchmesser des Lumens im Bereich zwischen dem distalen Ende und der ersten Seitenöffnung ca. um einen Faktor 2 - 2.5 mal grösser als der Durchmesser der ersten Seitenöffnung und/oder um einen Faktor 2 - 2.5 mal grösser als der minimale Innendurchmesser des ersten kanalartigen Bereichs. Im Vergleich mit dem Innendurchmesser des ersten Durchgangsbereichs ist der Innendurchmesser des Lumens im Bereich zwischen dem distalen Ende und der ersten Seitenöffnung insbesondere ca. um einen Faktor 1.75 - 2.25, bevorzugt um einen Faktor 1.8 - 2.0, grösser.

Prinzipiell kann das Lumen aber auch einen konstanten Innendurchmesser aufweisen. Wird dieser so gross gewählt, dass ein drahtförmige Element und der Führungsdraht nebeneinander Platz finden, leidet jedoch unter Umständen die Einführbarkeit des Katheters. Bei einem zu geringen Durchmesser findet lediglich ein einzelnes drahtförmiges Element im Lumen Platz, was der Flexibilität bei der Verwendung des Katheters entgegen steht.

Bevorzugt liegt am distalen Ende des Katheters eine verjüngte Katheterspitze vor. Damit kann die Einführbarkeit des Katheters weiter verbessert werden, da Engstellen in den Hohlorganen besser passierbar werden. Ein minimaler Innendurchmesser der Katheterspitze ist dabei insbesondere in etwa gleich dem Innendurchmesser des ersten Durchgangsbereichs. Respektive ist der Innendurchmesser des Lumens im Bereich zwischen der Katheterspitze und der ersten Seitenöffnung insbesondere ca. um einen Faktor 1.75 - 2.25, bevorzugt um einen Faktor 1.8 - 2.0, grösser als der minimale Innendurchmesser im Bereich der Katheterspitze. Eine derartig ausgebildete Katheterspitze kann im Zusammenspiel mit entsprechend dimensionierten drahtförmigen Elementen sicherstellen, dass immer nur ein einzelnes drahtförmiges Element durch die Katheterspitze geschoben werden kann.

In einer bevorzugten Ausführungsform ist die Katheterspitze durch einen, bevorzugt stufenartig, verjüngten Endabschnitt des Katheterschafts und ein stirnseitig und koaxial daran befestigtes hohlzylindrisches Rohrstück gebildet. Das hohlzylindrische Rohrstück weist insbesondere einen konstanten Innendurchmesser auf und ist bevorzugt aus einem weicheren Material gefertigt als der Katheterschaft. Besonders bevorzugt umfasst das hohlzylindrische Rohrstück ein proximales Teilstück, welches an den Katheterschaft angrenzt, sowie ein distales Teilstück, welches in distaler Richtung an das proximale Teilstück angrenzt und das distale Ende der Katheterspitze bildet. Mit anderen Worten ist das hohlzylindrische Rohrstück mit Vorteil zweiteilig ausgebildet. Das distale Teilstück besteht dabei mit Vorteil aus einem weicheren Material als das proximale Teilstück. Dies erhöht die Einführbarkeit des Katheters weiter, da die Katheterspitze in proximaler Richtung zunehmend härter wird. Das distale Teilstück kann z. B. durch eine stoffschlüssige Verbindungstechnik, insbesondere durch Verschweissung, mit dem proximalen Teilstück verbunden sein.

In einer besonders bevorzugten Ausführungsform weist das proximale Teilstück an seinem dem distale Teilstück zugewandten Ende einen Schrägschnitt auf, während das distale Teilstück an seinem dem proximalen Teilstück zugewandten Ende ebenfalls einen Schrägschnitt aufweist. Die Schrägschnitte von sind dabei insbesondere so ausgebildet, dass die beiden stirnseitig aneinander anliegenden Teilstücke eine gemeinsame Längsmittelachse aufweisen bzw. einen geraden hohlzylindrischen Körper bilden. Die Schrägschnitte weisen gegenüber einer Längsmittelachse des hohlzylindrischen Rohrstücks zudem bevorzugt einen Winkel von beispielsweise 20 - 50°, bevorzugt von ca. 25 - 35°, auf. Durch die Schrägschnitte wird der Übergang von einem weicheren distalen Teilstück zu einem härteren proximalen Teilstück insbesondere bezüglich der Längsrichtung sanfter ausgebildet, was der Einführbarkeit zu Gute kommt.

Es kann aber auch vorteilhaft sein, die Katheterspitze zum distalen Ende hin wenigstens abschnittsweise konisch verjüngend auszubilden. In diesem Fall können kann z. B. das distale und/oder das proximale Teilstück in Form eines Hohlkegelstumpfs vorliegen.

Es kann aber grundsätzlich auch ein einteiliges hohlzylindrisches Rohrstück verwendet werden oder gänzlich auf ein derartiges Röhrstück verzichtet werden, so dass die Katheterspitze z. B. lediglich aus einem verjüngten distalen Ende des Katheterschafts gebildet wird.

Prinzipiell kann auch vollständig auf eine Katheterspitze verzichtet werden, was zwar die Einführbarkeit vermindert, aber den Aufbau des Katheters vereinfacht.

Weiter bevorzugt liegt ein durch die zweite Seitenöffnung in das Lumen geführter Hilfsdraht vor, welcher bis in einen Bereich zwischen der ersten Seitenöffnung und dem distalen Ende hinein ragt, so dass insbesondere der neben dem ersten kanalförmigen Bereich liegende erste Durchgangsbereich des Lumens im Wesentlichen blockiert bzw. verschlossen ist. Durch den Hilfsdraht ist es insbesondere möglich, den ersten Durchgangsbereich im Wesentlichen zu verschliessen. Ein durch die vordere Öffnung in das Lumen eingeführtes drahtförmiges Element, z. B. ein Führungsdraht, welches insbesondere am distalen Ende des Hilfsdrahts vorbeigeschoben wird, wird dadurch automatisch in den ersten kanalartigen Bereich gelenkt und verlässt das Lumen durch die erste Seitenöffnung. Eine spezielle Biegung des Katheters ist hierfür nicht notwendig.

Dadurch dass der Hilfsdraht bis in den Bereich zwischen erster Seitenöffnung und distalem Ende hinein ragt, ist dieser ausreichend fixiert. Ein Herausrutschen ist daher nicht zu befürchten. Prinzipiell kann aber der Hilfsdraht z. B. auch kürzer ausgebildet sein, so dass er lediglich bis in den ersten kanalartigen Bereich hinein ragt. Dies ist aber weniger vorteilhaft, da ein von der vorderen Öffnung her kommendes drahtförmiges Element am relativ unbeweglichen Hilfsdraht so eher anstehen kann.

Grundsätzlich ist es ebenso möglich, den Hilfsdraht in die erste Seitenöffnung einzuführen. Dadurch lässt sich der erste Seitenausgang verschliessen und ein durch die vordere Öffnung in das Lumen eingeführtes drahtförmiges Element wird automatisch in den ersten Durchgangsbereich gelenkt.

Grundsätzlich ist es auch denkbar sowohl in der ersten als auch in der zweiten Seitenöffnung einen Hilfsdraht vorzusehen. In diesem Fall ragt der in der zweiten Seitenöffnung angeordnete Hilfsdraht jedoch mit Vorteil höchstens bis in den Bereich zwischen der ersten Seitenöffnung und der zweiten Seitenöffnung. Ein durch die vordere Öffnung in das Lumen eingeführtes drahtförmiges Element wird dadurch automatisch durch die Durchgangsbereiche zum proximalen Ende geleitet.

Mit Vorteil weist der Hilfsdraht eine ausserhalb der zweiten Seitenöffnung vorliegende Verdickung auf, welche als Anschlag dient und ein vollständiges Durchführen des Hilfsdrahts durch die zweite Seitenöffnung verhindert. Entsprechendes gilt natürlich auch bei einem in der ersten Seitenöffnung vorliegenden Hilfsdraht. Damit wird verhindert, dass der Hilfsdraht durch die Seitenöffnung hindurch in das Lumen hinein rutschen kann. Eine Verdickung lässt sich insbesondere relativ einfach anbringen, z. B. durch Aufschweissen von Kunststoff. Der Hilfsdraht ist insbesondere hauptsächlich aus Metall gefertigt.

Es sind aber zusätzlich oder anstelle der Verdickung auch andere Sicherungsmittel denkbar, so kann der Hilfsdraht an seinem aus der Seitenöffnung heraus ragenden Ende auch derart gebogen sein, dass eine Durchführung durch die zweite Seitenöffnung verunmöglicht wird und/oder der Hilfsdraht wird um die Aussenseite des Katheterschafts gewunden.

Besonders bevorzugt liegt in einem Bereich der ersten Seitenöffnung und/oder einem Bereich der zweiten Seitenöffnung eine Markierung am Katheterschaft vor, wobei die Markierung insbesondere als Farbmarkierung ausgebildet ist. Bei der Markierung kann es sich z. B. um ein an der Aussenseite des Katheterschafts eingeschweisstes und gefärbtes Kunststoffelement handeln.

Liegen zwei oder noch mehr Seitenöffnungen vor, sind die Markierungen insbesondere unterscheidbar ausgebildet. Damit sind die einzelnen und üblicherweise sehr kleinen Seitenöffnungen auf Anhieb erkennbar.

Mit Vorteil ist im Bereich des distalen Endes bzw. an der Katheterspitze ebenfalls wenigstens eine Markierung angebracht, wobei diese im Wesentlichen der an der ersten und/oder der zweiten Seitenöffnung angeordneten Markierungen entspricht. Damit werden die möglichen Wege der drahtförmigen Elemente im Katheterschaft verdeutlicht. Dabei ist es z. B. möglich ein distales Ende des Katheterschafts bzw. die Katheterspitze aus einem Material zu fertigen, welches eine der Markierung an der ersten und/oder der zweiten Seitenöffnung entsprechende Farbe aufweist.

Liegt eine mehrteilige Katheterspitze vor, welche z. B. ein proximales Teilstück und ein distales Teilstück umfasst, ist insbesondere das distale Teilstück aus einem Material gefertigt, welches eine der Markierung der ersten Seitenöffnung entsprechende Farbe aufweist. In diesem Fall ist das proximale Teilstück bevorzugt aus einem Material gefertigt, welches eine der zweiten Seitenöffnung entsprechende Farbe aufweist.

Falls zweckmässig können die Markierungen jedoch auch anders ausgebildet sein oder es kann gar ganz auf diese verzichtet werden. Dies kann allenfalls die Herstellung des Katheters vereinfachen.

In einem weiteren vorteilhaften Aspekt weist ein proximaler Abschnitt des Katheterschafts eine geringere Elastizität auf als ein distaler Abschnitt des Katheterschafts. Mit anderen Worten verfügt der proximale Abschnitt des Katheterschafts bevorzugt über ein grösseres Elastizitätsmodul als der distale Abschnitt des Katheterschafts. Dieser Aspekt kann auch unabhängig vom erfindungsgemässen Aufbau ganz allgemein bei Kathetern mit einem Katheterschaft vorteilhaft sein. Dadurch lässt sich die Einführbarkeit des Katheters weiter verbessern. Augrund des steiferen proximalen Abschnitts lässt sich der Katheter nämlich besser im Hohlorgan vorschieben, während der distale Abschnitt aufgrund der grösseren Flexibilität, dem Verlauf des Hohlorgans dennoch gut folgen kann. Grundsätzlich kann der Katheterschaft aber auch eine über die gesamte Länge im Wesentlichen konstant Elastizität aufweisen.

Besonders bevorzugt umfasst der proximale Abschnitt des Katheterschafts ein Metallröhrchen, während ein distaler Abschnitt ein Kunststoffröhrchen beinhaltet. Das Kunststoffröhrchen grenzt dabei insbesondere direkt an das Metallröhrchen an. Das Kunststoffröhrchen weist insbesondere eine höhere Elastizität auf als das Metallröhrchen. Durch die Kombination aus Metallröhrchen und Kunststoffröhrchen kann die Einführbarkeit des Katheters bei kompakterer Bauweise nochmals verbessert werden. Das Metallröhrchen gewährleistet dabei ein besonders wirksames Vorschieben des Katheters in einem Hohlorgan, während der distale Abschnitt mit dem Kunststoffröhrchen demgegenüber flexibel ist, so dass der Katheter dem Hohlorgan besonders gut folgen. Eine derartige Ausgestaltung ist aber nicht zwingend. Je nach Verwendungszweck des Katheters können auch andere Materialkombinationen oder ein einstückiger Katheterschaft vorteilhaft sein.

Ein Verhältnis der Länge des Metallröhrchens zur Länge des Kunststoffröhrchens beträgt bevorzugt 1.7 - 4.0. Eine Länge des Metallröhrchens misst insbesondere 1.1 - 1.2 m, während eine Länge des Kunststoffröhrchens 30 - 40 cm misst. Das Metallröhrchen besteht mit Vorteil aus Stahl, insbesondere nicht rostendem Stahl. Solche Kombinationen aus Metallröhrchen und Kunststoffröhrchen haben sich im Hinblick auf die Einführbarkeit und eine möglichst kompakte Bauweise des Katheters als optimal erwiesen. Es sind aber auch andere Kombinationen mit anders dimensionierten Röhrchen möglich.

Bevorzugt ist in einem Übergangsbereich zwischen dem proximalen Abschnitt und dem distalen Abschnitt des Katheterschafts ein Stützelement vorgesehen, welches so ausgebildet ist, dass ein Elastizitätssprung zwischen dem proximalen Abschnitt und dem distalen Abschnitt des Katheterschafts reduziert und/oder wenigstens teilweise ausgeglichen wird. Dadurch kann insbesondere die Knickgefahr am Übergang zwischen dem proximalen Abschnitt und dem distalen Abschnitt des Katheterschafts vermindert werden. Bevorzugt ist das Stützelement ein Stützdraht. Der Stützdraht verläuft insbesondere in longitudinaler Richtung des Katheterschafts. Ein Durchmesser des Stützdrahts beträgt insbesondere 0.10 - 0.50 mm, insbesondere 0.30 - 0.35 mm. Vorteilhafterweise besteht der Stützdraht aus Stahl, insbesondere nicht rostendem Stahl. Stahl hat sich aufgrund seiner Materialeigenschaften, insbesondere seiner Festigkeit und Flexibilität, als besonders geeignet für Übergangsdrähte herausgestellt. Je nach Material des Stützdrahts und gewünschter Flexibilität des Katheters können auch andere Dimensionen vorteilhaft sein.

Es ist auch möglich, anstelle eines Stützdrahts ein anderes Stützelement vorzusehen, z. B. ein Röhrchen oder ein blechartiges, flaches Element, welches insbesondere auch gebogen sein kann. Der Querschnitt des Stützelements muss daher nicht notwendigerweise rund sein, sondern kann durchaus auch Ecken beinhalten.

Das Stützelement oder der Stützdraht ist insbesondere am proximalen Abschnitt des Katheterschafts oder am Metallröhrchen angebracht, z. B. durch Verschweissung, und ragt in den Innenbereich des distalen Abschnitts des Kunststoffröhrchens hinein. Insbesondere entspricht eine Länge des vom proximalen Abschnitt oder dem Metallröhrchen abstehenden und in den distalen Abschnitt oder das Kunststoffröhrchen hinein ragenden Abschnitts des Stützdrahts 0.2 - 0.5 mal, bevorzugt 0.2 - 0.35 mal, der Gesamtlänge des Kunststoffröhrchens. Bei einem Kunststoffröhrchen mit einer Gesamtlänge von 30 - 40 cm misst in das Kunststoffröhrchen hinein ragenden Abschnitts des Stützdrahts beispielsweise 5 - 15 cm, bevorzugt 8 - 12 cm, weiter bevorzugt 9 - 11 cm. Damit kann ein optimaler Ausgleich des Elastizitätssprungs zwischen dem Metallröhrchen und dem Kunststoffröhrchen erreicht werden. Grundsätzlich kann der Stützdraht aber auch anders dimensioniert werden, sofern dies zweckdienlich erscheint.

Besonders vorteilhaft ist das Stützelement oder der Stützdraht derart am Metallröhrchen angebracht, dass ein innerer Hohlraum des Metallröhrchens vollständig frei bleibt. Dadurch kann der innere Hohlraum des Metallröhrchens bestmöglich genutzt werden, z. B. für die Durchführung von drahtförmigen Elementen. Prinzipiell kann das Stützelement oder der Stützdraht jedoch auch im Innern des Metallröhrchens angebracht werden. Damit wird aber unter Umständen die Durchführung von drahtförmigen Elementen behindert.

Zur Befestigung des Stützelements bzw. des Stützdrahts verfügt das Metallröhrchen mit Vorteil über einen in das Metallröhrchen eingebrachten Schlitz, welcher sich vom distalen Ende des Metallröhrchens in longitudinaler Richtung des Metallröhrchens erstreckt. Der Schlitz ist zur teilweisen Aufnahme des Stützelements bzw. Stützdrahts ausgebildet und weist bevorzugt eine Breite auf, welche einem Durchmesser und/oder einer maximalen Breite des Stützelements oder Stützdrahts entspricht. Damit kann das Stützelement bzw. der Stützdraht im Schlitz einfacher fixiert werden und ist zugleich zumindest teilweise in der Wand des Metallröhrchens eingelassen, was eine kompaktere Bauweise ermöglicht. Eine Länge des Schlitzes in longitudinaler Richtung entspricht mit Vorteil 0.01 - 0.05-mal der Gesamtlänge des Stützdrahts. In einer vorteilhaften Variante beträgt die Länge des Schlitzes 1 - 5 mm, bevorzugt 2.5 - 3.5 mm. Dadurch kann der Stützdraht optimal im Schlitz befestigt werden.

Es ist aber prinzipiell auch möglich, anstelle des Schlitzes lediglich eine von aussen in das Metallröhrchen eingebrachte Nut vorzusehen. Ebenso kann das Stützelement oder der Stützdraht auch ohne einen Schlitz oder eine Nut von aussen auf das Metallröhrchen aufgebracht werden.

Der Stützdraht verfügt insbesondere über einen kreisförmigen Querschnitt. In einer vorteilhaften Variante reduziert sich der Durchmesser zumindest in einem distalen Abschnitt des Stützdrahts in distaler Richtung. Insbesondere ist der Stützdraht zumindest in einem distalen Abschnitt konusförmig zulaufend ausgebildet. Damit kann die Elastizität des Stützdrahts an die spezifischen Erfordernisse angepasst werden, womit sich der Elastizitätssprung zwischen Metallröhrchen und Kunststoffröhrchen bestmöglich ausgleichen lässt. Eine Länge des konusförmig zulaufenden distalen Abschnitts beträgt bevorzugt 0.2 - 0.4-mal der Gesamtlänge des Stützdrahts, insbesondere 2 - 4 cm.

Bei einem Katheter mit wenigstens einer Seitenöffnung erstreckt sich das Stützelement oder der Stützdraht in distaler Richtung mit Vorteil maximal bis zum proximalen Bereich der wenigstens einen Seitenöffnung. Bei mehreren Seitenöffnungen erstreckt sich das Stützelement bzw. der Stützdraht in distaler Richtung insbesondere maximal bis zur hintersten Seitenöffnung, welche vom distalen Ende des Katheters am weitesten beabstandet ist. Insbesondere erstreckt sich das Stützelement oder der Stützdraht in distaler Richtung bis zur wenigstens einen Seitenöffnung oder bis zur hintersten Seitenöffnung. Damit wird gewährleistet, dass das Stützelement nicht in die Bereiche der wenigstens einen Seitenöffnung hineinragt und dabei die Einführung von drahtförmigen Elementen behindert. Gleichzeitig ist jedoch ein optimaler Ausgleich des Elastizitätssprungs zwischen Metallröhrchen und Kunststoffröhrchen gewährleistet. Insbesondere können drahtförmige Elemente somit auch über das proximale Ende des Metallröhrchen eingeführt und durch den Katheterschaft bzw. das Lumen geführt werden, was die Einsatzmöglichkeiten des erfindungsgemässen Katheters erweitert.

Bei Kathetern, welche im Bereich der wenigstens einen Seitenöffnung im Lumen über einen seitlichen und in die wenigstens eine Seitenöffnung mündenden kanalartigen Bereich verfügen, ist das Stützelement bzw. der Stützdraht mit Vorteil so ausgebildet, dass der neben dem kanalartigen Bereich vorliegende Durchgangsbereich im Lumen frei vollständig bleibt. Mit Vorteil erstreckt sich das Stützelement also maximal bis zum proximalen Ende des kanalartigen Durchgangsbereichs der wenigstens einen Seitenöffnung. Bei Seitenöffnungen entsprechend bis maximal zum proximalen Ende des kanalartigen Durchgangsbereichs der hintersten Seitenöffnung.

Grundsätzlich ist es aber auch möglich, das Stützelement beispielsweise bis in den Bereich der Katheterspitze ragen zu lassen, sofern dies für spezielle Anwendungen zweckdienlich ist. Allerdings werden dadurch allenfalls die Einsatzmöglichkeiten des Katheters vermindert.

Besonders vorteilhaft ist das Kunststoffröhrchen mit seinem proximalen Ende auf das distale Ende des Metallröhrchens aufgeschoben und am distale Ende des Metallröhrchens befestigt, z. B. durch Pressschweissung. Falls ein Stützelement, z. B. ein Stützdraht, angeordnet ist, umgibt das Kunststoffröhrchen das Stützelement mit Vorteil vollständig. Bei einer Befestigung des Stützelements bzw. des Stützdrahts mit einem Schlitz, wird das Kunststoffröhrchen insbesondere so angeordnet, dass der Schlitz vom Kunststoffröhrchen vollständig umgegeben ist. Dadurch sind die Hohlorgane beim Einführen des Katheters vor dem Stützelement bestmöglich geschützt, was die Einführbarkeit weiter verbessert.

Prinzipiell sind aber auch andere Anordnungen denkbar. So könnte das Kunststoffröhrchen z. B. mit Hilfe einer Hülse stirnseitig auf Stoss mit dem Metallröhrchen verbunden werden.

Mit Vorteil wird der erfindungsgemässe Katheter als Gerätesatz zusammen mit einem Führungsdraht sowie einen Spezialdraht für diagnostische, chirurgische und/oder therapeutische Zwecke eingesetzt. Damit kann der Katheter optimal auf den Führungsdraht und den Spezialdraht abgestimmt werden.

Ein Aussendurchmesser des Führungsdrahts und ein Aussendurchmesser des Spezialdrahts sind dabei mit Vorteil zusammen höchstens ungefähr gleich gross ist wie der Innendurchmesser des Lumens im Bereich zwischen dem distalen Ende bzw. der Katheterspitze und der ersten Seitenöffnung. Damit ist sichergestellt, dass der Führungsdraht und der Spezialdraht in diesem Bereich nebeneinander vorbei geschoben werden können. Um eine gute Verschiebbarkeit zu gewährleisten ist ein gewisses Übermass beim Innendurchmesser des Lumens im Bereich zwischen dem distalen Ende bzw. der Katheterspitze und der ersten Seitenöffnung empfehlenswert.

Es ist aber auch denkbar, den Innendurchmesser des Lumens im Bereich zwischen dem distalen Ende bzw. der Katheterspitze und der ersten Seitenöffnung deutlich grösser auszubilden als die beiden Aussendurchmesser der beiden Drähte zusammen. Im Hinblick auf möglichst geringe Abmessungen des Katheterschafts ist dies jedoch nachteilig.

Insbesondere entspricht ein Durchmesser der ersten Seitenöffnung dem Aussendurchmesser des Führungsdrahts, während ein Durchmesser der zweiten Seitenöffnung dem Aussendurchmesser des Spezialdrahts entspricht. Damit können die Durchmesser der beiden Seitenöffnungen so gering wie möglich gehalten werden, womit der Katheter insgesamt kompakter wird. Es kann aber auch vorteilhaft sein, die Durchmesser der ersten und der zweiten Seitenöffnung im Wesentlichen identisch und entsprechend dem dickeren der beiden Drähte auszubilden. Damit wird ein Maximum an Flexibilität bei der Verwendung des Gerätesatzes erreicht, da sowohl der Führungsdraht als auch der Spezialdraht in die erste und die zweite Seitenöffnung einführbar sind. Auch hier ist ein gewisses Übermass der Seitenöffnungen zu empfehlen.

Der Spezialdraht ist insbesondere als Sondendraht für ein tomographisches Verfahren ausgebildet, wobei es sich insbesondere um einen Sondendraht für eine optische Kohärenztomographie handelt. Es hat sich gezeigt, dass der erfindungsgemässe Katheter bzw. der erfindungsgemässe Gerätesatz insbesondere im Zusammenhang mit tomographischen Verfahren, im Besonderen in Blutbahnen, äusserst vorteilhaft sind. Gerade bei diesen Verfahren müssen oft hochviskose Kontrastmittel in Hohlorgane eingeleitet werden. Die Möglichkeit, hier neue und rationellere Verfahrensabläufe zu realisieren bringt grosse Vorteile mit sich.

Es sind aber grundsätzlich auch andere Spezialdrähte für diagnostische, therapeutische und/oder chirurgische Verfahren mit dem erfindungsgemässen Katheter verwendbar.

Der erfindungsgemässe Katheter bzw. ein Gerätesatz mit einem derartigen Katheter ist insbesondere geeignet zur Anwendung in diagnostischen, therapeutischen und/oder chirurgischen Verfahren in Blutbahnen, wobei es sich z. B. um Herzkranzgefässe, periphere Blutgefässe (in Armen und/oder Beinen) und/oder um Blutgefässe im Kopf handeln kann.

Bei einem vorteilhaften Verfahren, welches z. B. zu Diagnosezwecken in einer menschlichen und/oder einer tierischen Blutbahn durchgeführt wird, wird in einem ersten Schritt insbesondere ein drahtförmiges Element, z. B. ein in die erste Seitenöffnung eines erfindungsgemässen Katheters eingeführt.

### Kurze Beschreibung der Zeichnungen

Die zur Erläuterung des Ausführungsbeispiels verwendeten Zeichnungen zeigen:
- Fig. 1: einen Längsschnitt durch einen ersten Katheter mit einer einzigen Seitenöffnung;
- Fig. 2: einen Querschnitt durch den Katheter aus Fig. 1 entlang der Linie A - B;
- Fig. 3: einen Längsschnitt durch einen zweiten Katheter mit zwei beabstandeten und diametral gegenüberliegenden Seitenöffnungen, wobei der Katheter erfindungsgemäss ist;
- Fig. 4: einen Querschnitt durch den Katheter aus Fig. 3 entlang der Linie C - D;
- Fig. 5: den Katheter aus den Fig. 3 und 4 mit einem in die hintere Seitenöffnung eingebrachten Hilfsdraht;
- Fig. 6: den Katheter aus Fig. 5 auf einem durch die vordere Seitenöffnung verlaufenden Führungsdraht;
- Fig. 7: den Katheter aus Fig. 6 mit einem in die hintere Seitenöffnung eingeführten Sondendraht;
- Fig. 8: den Katheter aus Fig. 7, wobei der Führungsdraht in das Lumen des Katheters und der Sondendraht durch die Katheterspitze nach vorne geschoben wurde;
- Fig.9: einen Längsschnitt durch einen dritten Katheter mit einem zweiteiligen Katheterschaft umfassend ein Metallröhrchen und ein Kunststoffröhrchen, wobei im Übergangsbereich zwischen den beiden Röhrchen ein Stützelement zum Ausgleichen des Elastizitätssprungs angeordnet ist;
- Fig. 10: einen Querschnitt durch den Katheter aus Fig. 9 entlang der Linie E - F;
- Fig. 11: eine Aufsicht auf das Metallröhrchen des Katheters aus Fig.9 mit einem stirnseitig eingebrachten Schlitz zur Aufnahme des Stützelements;
- Fig. 12: eine Aufsicht auf das Stützelement des Katheters aus Fig. 9;

Grundsätzlich sind in den Figuren gleiche Teile mit gleichen Bezugszeichen versehen.

### Wege zur Ausführung der Erfindung

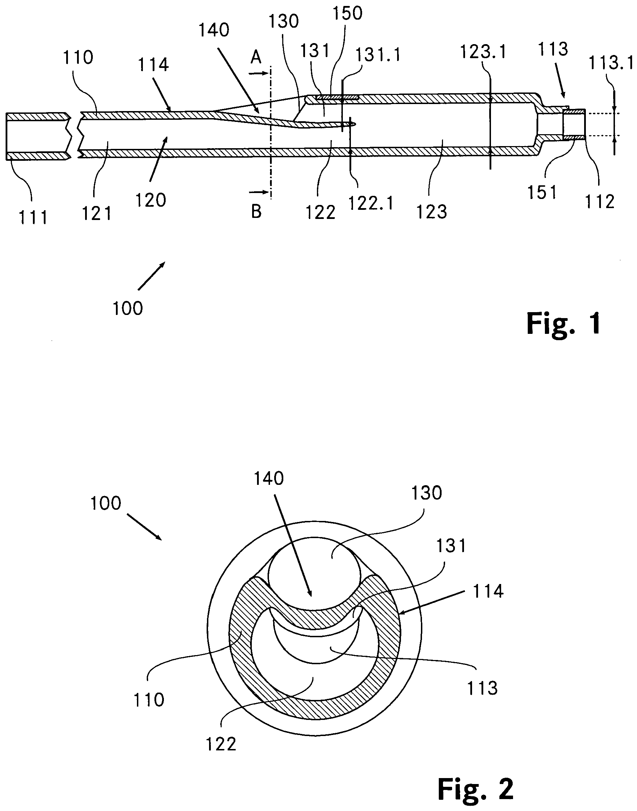

In den Fig. 1 und 2 ist ein erster Katheter 100 in einem Längs- und einem Querschnitt dargestellt. Bei dieser Ausführungsform ist zwar nur eine Seitenöffnung vorgesehen, was nicht erfindungsgemäss ist. Was die sonstigen Einzelheiten betrifft, dient die Ausführungsform aber zur Erläuterung und zum Verständnis der Erfindung. Der erste Katheter 100 ist ein Einlumenkatheter und umfasst einen Katheterschaft 110 in Form eines im Wesentlichen hohlzylindrischen Röhrchens. Der Katheterschaft 110 besteht z. B. aus einem an sich bei Kathetern bekannten Kunststoff. Ein Lumen 120 bzw. ein innerer und im Wesentlichen zylindrischer Hohlraum des Katheterschafts 110 erstreckt sich dabei vom proximalen Ende 111 des Katheterschafts bzw. des hohlzylindrischen Röhrchens zu seinem distalen Ende 112. Im Bereich des distalen Endes 112 ist der Katheterschafts 110 stufenartig zu einer Katheterspitze 113 verjüngt. Die Katheterspitze 113 weist dabei am distalen Ende ein koaxial angeordnetes hohlzylindrisches Rohrstück 151 mit konstantem Innendurchmesser auf, welches aus einem weicheren Material gefertigt ist als der Katheterschaft 110 und beispielsweise durch Druckschweissung angebracht ist. Das hohlzylindrische Rohrstück 151 weist zudem z. B. eine rote Farbe auf und dient zusätzlich als Markierung.

In einem Bereich, welcher z. B. ca. 5 - 15 cm hinter dem distalen Ende 112 liegt, ist an der Aussenseite 114 eine ungefähr kreisrunde Seitenöffnung 130 seitlich in den Katheterschaft 110 bzw. das hohlyzylindrische Röhrchen eingebracht. Die Seitenöffnung 130 zeigt dabei zum proximalen Ende 111 hin. Ein Normalenvektor der Öffnungsfläche steht somit schräg zur Längsachse des Kathterschafts 110. Die Seitenöffnung 130 mündet über einen in eine Richtung zum distalen Ende 112 hin und ungefähr parallel zur Längsachse des Lumens 120 verlaufenden kanalartigen Bereich 131 in einen hinter dem distalen Ende 112 liegenden distalen Bereich 123 des Lumens 120. Der kanalartige Bereich 131 ist an einer Seitenfläche des Lumens 120 eingebracht und weist einen zylindrischen Hohlraum mit einem über die Länge im Wesentlichen konstanten Innendurchmesser 131.1 auf. Der Innendurchmesser 131.1 des kanalartigen Bereichs 131 entspricht dabei ungefähr dem Durchmesser der Seitenöffnung 130. Neben dem kanalartigen Bereich 131 liegt ein Durchgangsbereich 122 im Lumen 120 mit einem minimalen Innendurchmesser 122.1 vor, so dass ein in proximaler Richtung an die Seitenöffnung 130 anschliessender proximaler Bereich 121 des Lumens 120 mit dem distalen Bereich 123 vor der Seitenöffnung 130 und vor dem kanalartigen Bereich 131 kommuniziert.

In einem in Längsrichtung an die Seitenöffnung 130 angrenzenden dem proximalen Ende 111 zugewandten Bereich der Aussenseite 114 ist zudem eine Führungsrinne 140 als Einführvorrichtung für ein drahtförmiges Element in die Aussenseite 114 des Katheterschafts 110 eingeformt. Die Führungsrinne 140 ist als konkav gewölbte oder wannenförmige Einbuchtung im Katheterschaft 110 ausgebildet und weist eine in Richtung auf die Seitenöffnung 130 hin zunehmende Tiefe auf. Der Übergang zwischen der Führungsrinne 140 und der Seitenöffnung 130 sowie der Übergang zwischen der Führungsrinne 140 und dem an die Führungsrinne 140 anschliessenden Bereich der Aussenseite 114 ist dabei naht- und stufenlos ausgebildet.

In einem dem distalen Ende 112 zugewandten und an die Seitenöffnung 130 angrenzenden Bereich ist eine Markierung 150 in Form eines beispielsweise roten Farbsegments aus Kunststoff im Katheterschaft 110 eingebracht. Die Markierung 150 bzw. das rote Farbsegment ist z. B. durch Druckschweissung angebracht, so dass weder Kanten, Vorstände noch Nähte vorliegen. Die Farbe der Markierung 150 und die Farbe des hohlzylindrisches Rohrstücks 151, welches ebenfalls als Markierung dient, sind im Wesentlichen identisch.

Ein minimaler Innendurchmesser 113.1 der Katheterspitze 113 misst z. B. ca. 0.51 mm, während der distale Bereich 123 des Lumens 120, bzw. der Bereich zwischen der Seitenöffnung 130 und der Katheterspitze 113, einen Innendurchmesser 123.1 von beispielsweise ca. 0.92 mm aufweist. Der Innendurchmesser 131.1 des kanalartigen Bereichs 131 misst z. B. ca. 0.41 mm, während der minimale Innendurchmesser 122.1 des Durchgangsbereichs 122 z. B. ca. 0.51 mm beträgt. Im proximalen Bereich 121 des Lumens 120 beträgt der Innendurchmesser beispielsweise mindestens ca. 0.52 mm. Eine Wandstärke des Katheterschafts 110 misst beispielsweise ungefähr 0.08 - 0.12 mm. Der Innendurchmesser 131.1 des kanalartigen Bereichs 130 und der minimale Innendurchmesser 122.1 des Durchgangsbereichs 122 sind zusammen in etwa gleich gross wie der Innendurchmesser 123.1 des distalen Bereichs 123.

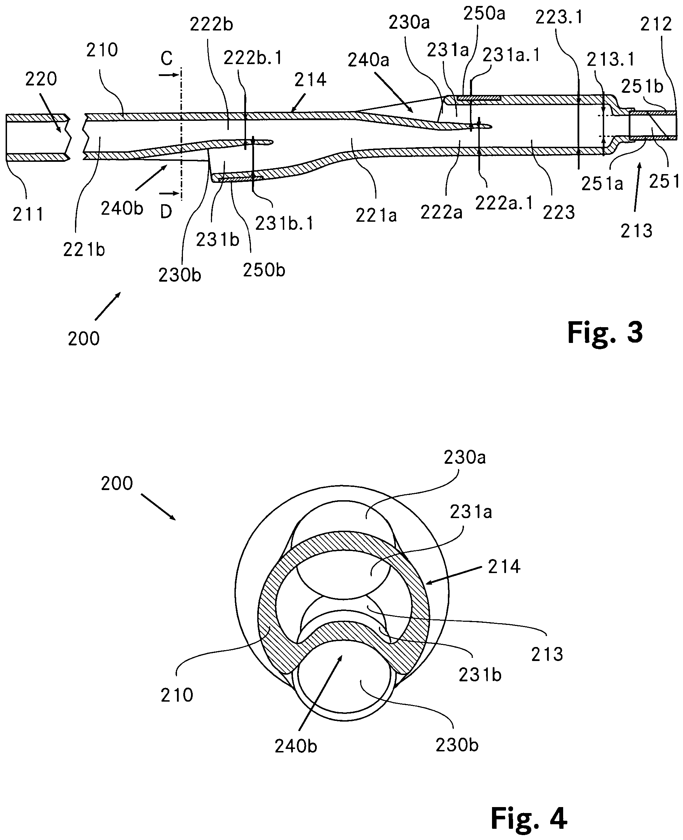

Die Fig. 3 und 4 zeigen einen zweiten Katheter 200 in einem Längs- und einem Querschnitt. Der Katheter ist erfindungsgemäss. Wie der erste Katheter 100 ist auch der zweite Katheter 200 ein Einlumenkatheter und umfasst einen Katheterschaft 210 in Form eines im Wesentlichen hohlzylindrischen Röhrchens. Der Katheterschaft 210 besteht z. B. aus einem an sich bei Kathetern bekannten Kunststoff. Ein Lumen 220 bzw. ein innerer und im Wesentlichen zylindrischer Hohlraum des Katheterschafts 210 erstreckt sich dabei vom proximalen Ende 211 des Kathterschafts bzw. des hohlzylindrischen Röhrchens zu seinem distalen Ende 212. Im Bereich des distalen Endes 212 ist der Katheterschaft 210 stufenartig zu einer Katheterspitze 213 verjüngt. Die Katheterspitze 213 weist dabei am distalen Ende ein koaxial angeordnetes hohlzylindrisches Rohrstück 251 mit konstantem Innendurchmesser auf. Das hohlzylindrische Rohrstück 251 weist dabei ein stirnseitig mit dem verjüngten Ende des Katheterschafts 210 verschweisstes proximales Teilstück 251a auf. Ein distales Ende des proximalen Teilstücks 251a ist schräg, bzw. unter einen Winkel von ca. 30°, zur Längsachse des Katheters ausgebildet. Mit anderen Worten weist das proximale Teilstück 251a an seinem distalen Ende einen Schrägschnitt auf. An dem distalen Ende des proximalen Teilstücks 251a liegt koaxial ein distales Teilstück 251b des hohlzylindrischen Rohrstücks 251 vor. Das proximale Ende des distalen Teilstücks 251b ist dabei ebenfalls schräg, bzw. unter einen Winkel von ca. 30°, ausgebildet. Die beiden Teilstücke sind stoffschlüssig miteinander verschweisst.

Das distale Teilstück 251b ist aus einem weicheren Material gefertigt als das proximale Teilstück 251b, welches wiederum aus einem weicheren Material gefertigt ist als der Katheterschaft 210. Das Material des distalen Teilstücks 251b weist zudem beispielsweise eine rote Farbe auf, welche Markierung dient. Das Material des proximalen Teilstücks 251a weist z. B. eine schwarze Farbe auf, welche ebenfalls als Markierung dient.

In einem Bereich, welcher z. B. ca. 5 - 15 cm hinter dem distalen Ende 212 liegt, ist an der Aussenseite 214 eine ungefähr kreisrunde erste Seitenöffnung 230a seitlich in den Katheterschaft 210 bzw. das hohlyzylindrische Röhrchen eingebracht. Die erste Seitenöffnung 230a zeigt dabei zum proximalen Ende 211 hin. Ein Normalenvektor der Öffnungsfläche steht somit schräg zur Längsachse des Kathterschafts 210. Die erste Seitenöffnung 230a mündet über einen in eine Richtung zum distalen Ende 212 hin und ungefähr parallel zur Längsachse des Lumens 220 verlaufenden ersten kanalartigen Bereich 231a in einen hinter dem distalen Ende 212 liegenden distalen Bereich 223 des Lumens 220. Der erste kanalartige Bereich 231a ist an einer Seitenfläche des Lumens 220 eingebracht und weist einen zylindrischen Hohlraum mit einem über die Länge im Wesentlichen konstanten Innendurchmesser 231a.1 auf. Der Innendurchmesser 231a.1 des ersten kanalartigen Bereichs 231a entspricht dabei ungefähr dem Durchmesser der ersten Seitenöffnung 230a. Neben dem ersten kanalartigen Bereich 231a liegt ein erster Durchgangsbereich 222a im Lumen 220 mit einem minimalen Innendurchmesser 222a.1 vor.

In einem in Längsrichtung an die erste Seitenöffnung 230a angrenzenden dem proximalen Ende 211 zugewandten Bereich der Aussenseite 214 ist zudem eine erste Führungsrinne 240a als Einführvorrichtung für ein drahtförmiges Element in die Aussenseite 214 des Katheterschafts 210 eingeformt. Die erste Führungsrinne 240a ist als konkav gewölbte oder wannenförmige Einbuchtung im Katheterschaft 210 ausgebildet und weist eine in Richtung auf die erste Seitenöffnung 230a hin zunehmende Tiefe auf. Der Übergang zwischen der ersten Führungsrinne 240a und der ersten Seitenöffnung 230a sowie der Übergang zwischen der ersten Führungsrinne 240a und dem an die erste Führungsrinne 240a anschliessenden Bereich der Aussenseite 214 ist dabei naht- und stufenlos ausgebildet.

In einem Bereich, welcher in proximaler Richtung z. B. ca. 2 - 5 cm von der ersten Seitenöffnung 240a beabstandet ist, ist diametral gegenüberliegend zur ersten Seitenöffnung 240a an der Aussenseite 214 eine ungefähr kreisrunde zweite Seitenöffnung 230b seitlich in den Katheterschaft 210 bzw. das hohlzylindrische Röhrchen eingebracht. Die zweite Seitenöffnung 230b zeigt dabei ebenfalls zum proximalen Ende 211 hin. Ein Normalenvektor der Öffnungsfläche steht somit schräg zur Längsachse des Kathterschafts 210. Die zweite Seitenöffnung 230b mündet über einen in eine Richtung zum distalen Ende 212 hin und ungefähr parallel zur Längsachse des Lumens 220 verlaufenden zweiten kanalartigen Bereich 231b in einen Bereich 221a zwischen der ersten Seitenöffnung 240a und der zweiten Seitenöffnung 240b des Lumens 220. Der zweite kanalartige Bereich 231b ist an einer dem ersten kanalartigen Bereich 231a gegenüberliegenden Seitenfläche des Lumens 220 eingebracht und weist einen zylindrischen Hohlraum mit einem über die Länge im Wesentlichen konstanten Innendurchmesser 231b.1 auf. Der Innendurchmesser 231b.1 des zweiten kanalartigen Bereichs 231b entspricht dabei ungefähr dem Durchmesser der zweiten Seitenöffnung 230b. Neben dem zweiten kanalartigen Bereich 231b liegt im Lumen 220 ein zweiter Durchgangsbereich 222b mit einem minimalen Innendurchmesser 222b.1 vor, so dass ein in proximaler Richtung an die zweite Seitenöffnung 230b anschliessender proximaler Bereich 221b des Lumens 220 mit dem Bereich 221a zwischen der ersten Seitenöffnung 240a und der zweiten Seitenöffnung 240b kommuniziert. Aufgrund des ersten Durchgangsbereichs 222a besteht damit zwischen dem proximalen Bereich 221b und dem distalen Bereich 223 vor der ersten Seitenöffnung 240a bzw. vor dem ersten kanalartigen Bereich 230a eine durchgängige Verbindung.

In einem in Längsrichtung an die zweite Seitenöffnung 230b angrenzenden dem proximalen Ende 211 zugewandten Bereich der Aussenseite 214 ist zudem eine zweite Führungsrinne 240b als Einführvorrichtung für ein drahtförmiges Element in die Aussenseite 214 des Katheterschafts 210 eingeformt. Die zweite Führungsrinne 240b ist als konkav gewölbte oder wannenförmige Einbuchtung im Katheterschaft 210 ausgebildet und weist eine in Richtung auf die zweite Seitenöffnung 230b hin zunehmende Tiefe auf. Der Übergang zwischen der zweiten Führungsrinne 240b und der zweiten Seitenöffnung 230b sowie der Übergang zwischen der zweiten Führungsrinne 240b und dem an die zweite Führungsrinne 240b anschliessenden Bereich der Aussenseite 214 ist dabei naht- und stufenlos ausgebildet.

In einem dem distalen Ende 112 zugewandten und an die erste Seitenöffnung 230a angrenzenden Bereich ist eine erste Markierung 250a in Form eines beispielsweise roten Farbsegments aus Kunststoff im Katheterschaft 210 eingebracht. Ebenso ist in einem dem distalen Ende 112 zugewandten und an die zweite Seitenöffnung 230b angrenzenden Bereich eine zweite Markierung 250b in Form eines beispielsweise schwarzen Farbsegments aus Kunststoff im Katheterschaft 210 eingebracht.

Die Farbe der ersten Markierung 250a und die Farbe des distalen Teilstücks 251a sind dabei im Wesentlichen identisch, weisen also z. B. die gleiche rote Farbe auf, um den Durchgang von der ersten Seitenöffnung 230a zur Katheterspitze 213 zu kennzeichnen. Die Farbe der zweiten Markierung 250b und Farbe des proximalen Teilstücks 251b sind ebenfalls im Wesentlichen identisch, weisen also z. B. die gleiche schwarze Farbe auf, um den Durchgang von der zweiten Seitenöffnung 230b zur Katheterspitze 213 zu kennzeichnen. Die beiden Markierungen 250a 250b bzw. die Farbsegmente sind z. B. durch Druckschweissung angebracht, so dass weder Kanten, Vorstände noch Nähte vorliegen.

Ein minimaler Innendurchmesser 213.1 der Katheterspitze 213 misst z. B. ca. 0.51 mm, während der distale Bereich 223 des Lumens 220, bzw. der Bereich zwischen der ersten Seitenöffnung 230a und der Katheterspitze 213, einen Innendurchmesser 223.1 von beispielsweise ca. 0.92 mm aufweist. Der Innendurchmesser 231a.1 des ersten kanalartigen Bereichs 231a misst z. B. ca. 0.41 mm, während der minimale Innendurchmesser 222a.1 des ersten Durchgangsbereichs 222a z. B. ca. 0.51 mm beträgt. Im Bereich 221a zwischen der ersten Seitenöffnung 230a und der zweiten Seitenöffnung 230b beträgt der Innendurchmesser des Lumens 220 beispielsweise mindestens ca. 0.52 mm.

Der Innendurchmesser 231b.1 des zweiten kanalartigen Bereichs 231b misst z. B. ca. 0.41 mm, während der minimale Innendurchmesser 222a.1 des zweiten Durchgangsbereichs 222a z. B. ca. 0.51 mm beträgt. Der an die zweite Seitenöffnung 230b anschliessende proximale Bereich 221b des Lumens 220 weist einen Innendurchmesser von beispielsweise mindestens ca. 0.52 mm auf.

Der Innendurchmesser 231b.1 des zweiten kanalartigen Bereichs 231b und der minimale Innendurchmesser 222b.1 des zweiten Durchgangsbereichs 222b sind zusammen ebenfalls in etwa gleich gross wie der Innendurchmesser 223.1 des distalen Bereichs 223. Eine Wandstärke des Katheterschafts 210 misst beispielsweise ungefähr 0.08 - 0.12 mm.

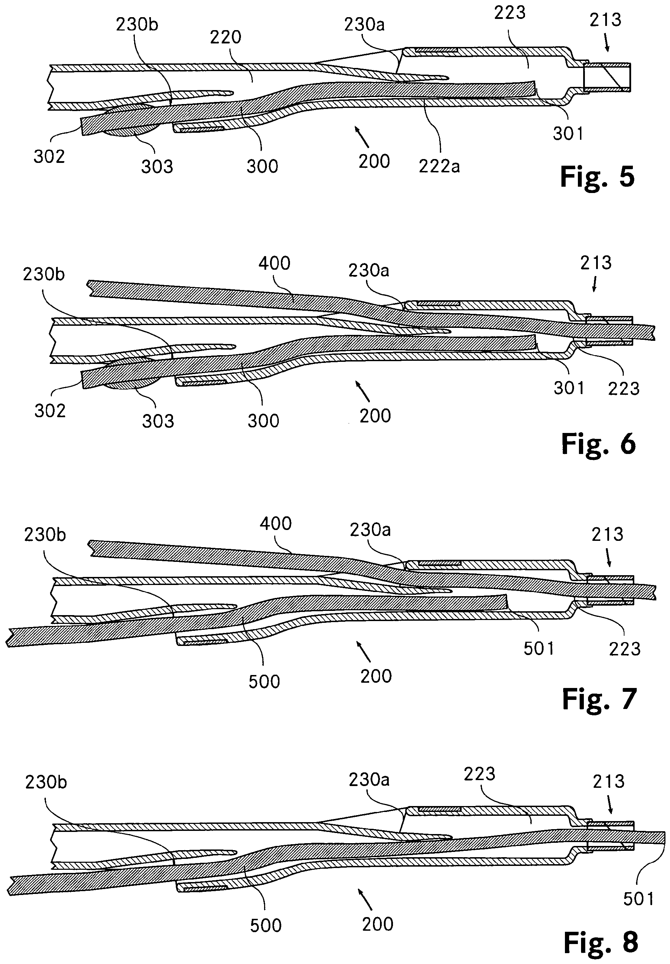

Fig. 5 zeigt den zweiten Katheter 200 aus den Fig. 3 und 4 mit einem von aussen in die zweite Seitenöffnung 230b eingeführten Hilfsdraht 300. Der Hilfsdraht ist dabei durch den ersten Durchgangsbereich 222a durchgeführt und ein vorderes Ende 301 des Hilfsdrahts 300 liegt im distalen Bereich 223 des Lumens hinter der Katheterspitze 213. Am hinteren und ausserhalb des Katheterschafts liegenden Ende 302 des Hilfsdrahts 300 ist eine Verdickung 303 angeordnet. Die Verdickung weist dabei einen minimalen Durchmesser auf, welcher grösser ist als ein Durchmesser der zweiten Seitenöffnung 230b, so dass ein vollständiges Hineinrutschen des Hilfsdrahts 300 in das Lumen 220 verunmöglicht wird. Ein Durchmesser des Hilfsdrahts entspricht idealerweise ungefähr dem minimalen Innendurchmesser 222a.1 des ersten Durchgangsbereichs 222a, so dass dieser im Wesentlichen verschlossen bzw. für die Durchführung von weiteren drahtförmigen Elementen blockiert ist.

In den Fig. 6 - 8 wird eine vorteilhafte Verwendung des in Fig. 5 gezeigten Katheters 200 mit eingeführtem Hilfsdraht 500 dargestellt. Dabei werden folgende Verfahrensschritte vorgenommen:
In einem ersten Verfahrensschritt wird der in Fig. 5 gezeigte Katheter 200 mit einem in die zweite Seitenöffnung 230b eingeführten Hilfsdraht 500 bereit gestellt. Der zweite Katheter 200 ist dabei mit Vorteil Bestandteil eines Gerätesatzes, welcher neben dem zweiten Katheter 200 auch einen Führungsdraht 400 und einem Sondendraht 500 umfasst.

In einem zweiten Verfahrenschritt wird durch die vordere Öffnung bzw. die Katheterspitze 213 das hintere Ende eines Führungsdrahts 400, welcher mit seinem vorderen Ende beispielsweise bereits in einer Blutbahn positioniert wurde, eingefädelt und in den distalen Bereich 223 geschoben. Der Führungsdraht weist z. B. einen Durchmesser von ca. 0.36 mm auf. Das vordere Ende wird nun am Hilfsdraht 300 vorbei geschoben und dabei automatisch in den ersten kanalartigen Bereich 231a gelenkt und von dort durch die erste Seitenöffnung 230a aus dem Lumen 220 heraus geführt. Die nun vorliegende Situation ist in Fig. 6 dargestellt.

Anschliessend wird in einem dritten Verfahrenschritt der Hilfsdraht 300 aus der zweiten Seitenöffnung 230b herausgezogen und entfernt.

In einem darauf folgenden vierten Verfahrensschritt wird nun mit Hilfe der zweiten Führungsrinne 240b ein drahtförmiges Element in Form eines Sondendrahts 500 für optische Kohärenztomographie in die zweite Seitenöffnung 230b eingeschoben, bis das vordere Ende 501 des Sondendrahts im distalen Bereich 223 des Lumens 220 neben dem Führungsdraht 400 zu liegen kommt. Diese Situation ist in Fig. 7 dargestellt. Der Sondendraht weist z. B. einen Durchmesser von ca. 0.49 mm auf.

Darauf hin wird der zweite Katheter 200 im fünften Verfahrensschritt entlang dem Führungsdraht 400 durch einen allenfalls vorhandenen Herzkranzkatheter hindurch in die gewünschte Position in der Blutbahn geschoben. Der Sondendraht behält dabei seine relative Position im Lumen 220 des zweiten Katheters 200.

In einem sechsten Verfahrensschritt wird der Führungsdraht 400 zurückgezogen, so dass das vordere Ende 401 des Führungsdrahts im distalen Bereich 223 hinter der Katheterspitze 213 bzw. zwischen erster Seitenöffnung 230b und Katheterspitze 213 zu liegen kommt. Alternativ kann der Führungsdraht 400 auch vollständig aus dem Katheter 200 und/oder der Blutbahn entfernt werden.

Daraufhin wird im siebten Verfahrensschritt der Sondendraht 500 nach vorne aus der Katheterspitze 213 heraus geschoben. Diese Situation entspricht Fig. 8
Der zweite Katheter 200 kann nun im achten Verfahrenschritt entlang dem Sondendraht 500 vollständig aus der Blutbahn und einem allfälligen Herzkranzkatheter herausgezogen werden. Somit verbleibt einzig der Sondendraht 500 in der Blutbahn. Damit können auch z. B. hochviskose Kontrastmittel problemlos in die Blutbahn eingeleitet werden. Der Zugang durch einen allenfalls vorhandenen Herzkranzkatheter wird durch den darin befindlichen Sondendraht 500 kaum beeinträchtigt.

In einem neunten Verfahrensschritt kann der Sondendraht 500 nach erfolgter Behandlung vollständig zurückgezogen werden. Alternativ kann der zweite Katheter 200 nach erfolgter Behandlung aber auch wieder entlang dem Sondendraht 500, welcher jetzt als Führungsdraht für den Katheter wirkt, nach vorne in die Blutbahn geschoben werden. Anschliessend kann z. B. der Sondendraht 500 in den distalen Bereich 223 zurück gezogen werden und der Führungsdraht 400 wird wieder aus der Katheterspitze 213 heraus geschoben, so dass dieser beispielsweise wieder in der ursprünglichen Position zu liegen kommt. Damit liegt im Wesentlichen wieder liegt die in Fig. 8 gezeigte Situation vor.

Es kann dann in einem allfälligen weiteren Verfahrensschritt der zweite Katheter 200 mit dem Sondendraht 500 entlang dem Führungsdraht 400 zurück gezogen werden.

Der zweite Katheter 200 kann nun falls erwünscht beispielsweise mit einem weiteren drahtförmigen Element bestückt werden und entsprechen kann wieder beim zweiten Verfahrensschritt begonnen werden.

Liegt lediglich eine Seitenöffnung vor, wie dies beim ersten Katheter 100 der Fall ist, kann z. B. folgendes Verfahren durchgeführt werden:

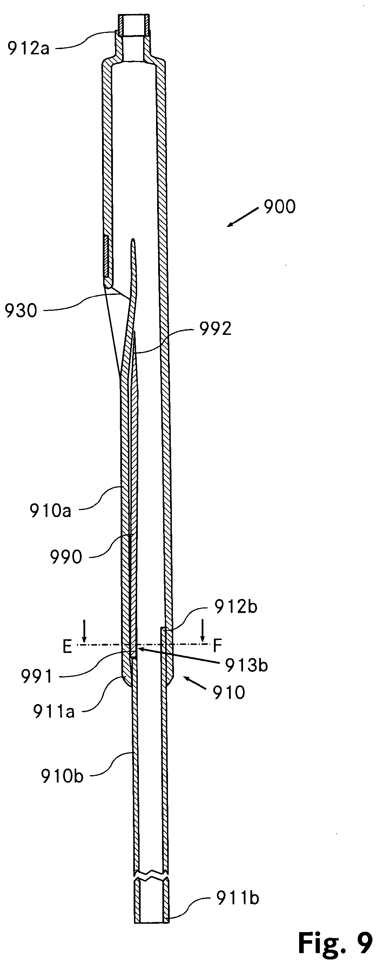

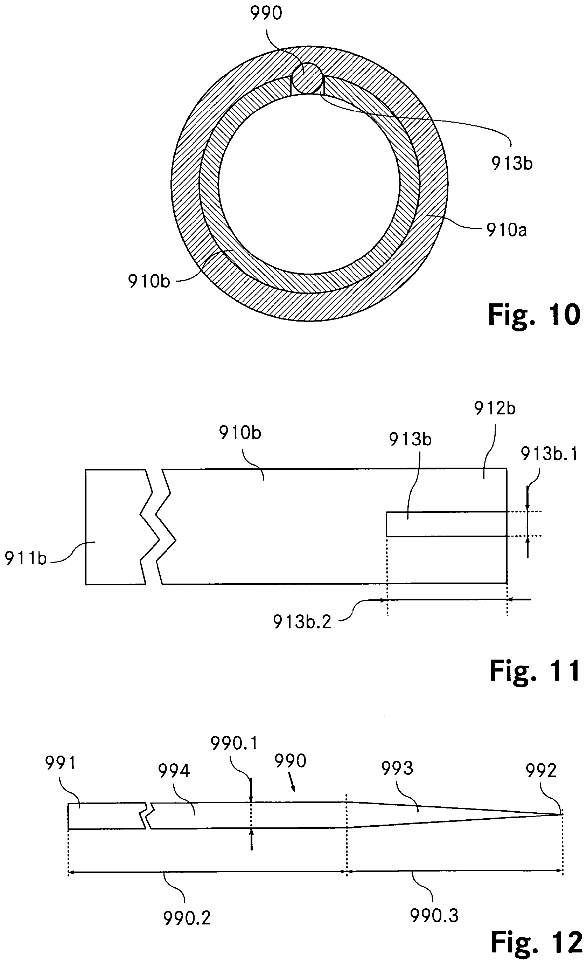

Die Fig. 9 umfasst einen dritten Katheter 900 in einem Längschnitt entlang der longitudinalen Achse. Fig. 10 stellt einen Querschnitt durch den drittenKatheter 900 entlang der Linie E - F aus Fig. 9 dar. Bis auf den Katheterschaft 910 ist der dritte Katheter 900 im Wesentlichen baugleich mit dem ersten Katheter 100 aus den Fig. 1 und 2.

Der Katheterschaft 910 des dritten Katheters 900 besteht aus einem distalen Abschnitt aus einem hohlzylindrischen Kunststoffröhrchen 910a und einem daran anschliessenden proximalen Abschnitt aus einem hohlzylindrischen Metallröhrchen 910b aus nicht rostendem Stahl.

Am distalen Ende 912b des Metallröhrchens 910b ist ein Schlitz 913b in das Metallröhrchen 910b eingebracht. Der Schlitz 913b erstreckt sich dabei von der Stirnseite am distalen Ende 912b in longitudinaler Richtung des Metallröhrchens 910b. Im Schlitz 913b ist das proximale Ende 991 eines Stützdrahts 990 angeordnet und mit dem Metallröhrchen durch Laserschweissung punktuell stoffschlüssig verbunden. Der Stützdraht 990 wirkt dabei als Stützelement. Der Stützdraht 990 ragt in distaler Richtung in das Kunststoffröhrchen 910a und reduziert den Elastizitätsprung zwischen Metallröhrchen 910b und Kunststoffröhrchen 910a, so dass die Einführbarkeit des Katheters 900 in ein Hohlorgan verbessert wird. Das distale Ende 992 des Stützdrahts 990 liegt in distaler Richtung unmittelbar vor der Seitenöffnung 130. Mit anderen Worten ragt der Stützdraht 990 bis zum proximalen Bereich der wenigstens einen Seitenöffnung 130.

Der in das Kunststoffröhrchen ragende Abschnitt des Stützdrahts 990 entspricht ca. 0.2 - 0.3-mal der Gesamtlänge des Kunststoffröhrchens 910a, wobei das Kunststoffröhrchen 910a eine Gesamtlänge von 30 - 40 cm aufweist, Die Gesamtlänge des Kunststoffröhrchens 910a wird dabei gemessen vom proximalen Ende 911a bis zum distalen Ende 912a. Eine Gesamtlänge des Metallröhrchens 910b misst z. B. 1.1 - 1.2 m.

Das proximale Ende 911a des Kunststoffröhrchens 910a ist über das distale Ende 912b des Metallröhrchens 910b geschoben und durch Pressschweissung mit diesem verbunden. Dabei ist das proximale Ende 911a in proximaler Richtung auch über den Schlitz 913b und den darin aufgenommenen und befestigten Stützdraht 990 geschoben, so dass eine fluiddichte Verbindung zwischen den Kunststoffröhrchen 910a und dem Metallröhrchen 910b vorliegt.

Zur Verdeutlichung des Aufbaus zeigt Fig. 11 eine Aufsicht auf das Metallröhrchen 910b vor dem Zusammenbau des dritten Katheters 900. Der Schlitz 913b zur Aufnahme Stützdrahts 990 weist in longitudinaler Richtung gemessen eine Länge 913b.2 von ca. 3 mm auf. Eine Breite des Schlitzes (gemessen senkrecht zur longitudinalen Richtung) beträgt ca. 0.33 mm.

Fig. 12 zeigt entsprechend den Stützdraht 990 vor dem Zusammenbau des Katheters 900. Der Stützdraht 990 weist einen zum distalen Ende 992 hin konusförmig zulaufenden Abschnitt 993 auf. Eine Länge des konusförmig zulaufenden Abschnitts 993 beträgt ca. 3 cm. In proximaer Richtung schliesst ein zylindrischer Abschnitt 994 mit kreisförmigem Querschnitt und einem konstanten Durchmesser 990.1 von ca. 0.33 mm an den konusförmig zulaufenden Abschnitts 993 an, welcher sich bis zum proximalen Ende 991 des Stützdrahts 990 erstreckt. Eine Länge 990. 2 des zylindrischen Abschnitts 994 beträgt ca. 7 cm. Eine Gesamtlänge des Stützdrahts 990, also die Länge 990.2 des zylindrischen Abschnitts 994 plus die Länge 990.3 des konusförmig zulaufenden Abschnitts 993, misst als ca. 10 cm. Entsprechend entspricht die Länge 990.3 des konusförmig zulaufenden distalen Abschnitts 993 in etwa 0.3-mal der Gesamtlänge des Stützdrahts 990.

Die Länge 913b. 2 des Schlitzes 913 in longitudinaler Richtung entspricht ca. 0.03 mal der Gesamtlänge des Stützdrahts 990, während die Breite 913b.1 des Schlitzes 913b ungefähr dem Durchmesser 990.1 des Stützdrahts 990 im zylindrischen Abschnitt 994 entspricht.

Der erste Katheter 100 aus den Fig. 1 und 2 lässt sich z. B. folgendermassen einsetzen:
Ein drahtförmiges Element, z. B. ein Sondendraht für optische Kohärenztomographie, wird ausserhalb des menschlichen und/oder tierischen Körpers von aussen durch die Seitenöffnung 130 des ersten Katheters 100 in das Lumen 220 eingeführt und in den Bereich 123 zwischen Seitenöffnung 130 und distalem Ende 112 eingeschoben.

An der vorderen Öffnung am distalen Ende 112 bzw. an der Katheterspitze 113 wird der erste Katheter 100 auf das proximale Ende eines bereits im Hohlorgan positionierten Führungsdrahts aufgeschoben.

Der erste Katheter 100 wird nun entlang dem Führungsdraht in das Hohlorgan geschoben und in Position gebracht, wobei der Führungsdraht im Lumen 120 am drahtförmigen Element und der Seitenöffnung 130 vorbei durch den Durchgangsbereich 122 in Richtung zum proximalen Ende 111 des Katheterschafts geschoben wird. Der distale Bereich 123 ist dabei insbesondere so dimensioniert, so dass der Führungsdraht und das drahtförmige Element nebeneinander Platz finden und gegeneinander verschoben werden können. Während der Bewegung entlang dem Führungsdraht behält das drahtförmige Element dabei seine Position relativ zur Seitenöffnung 130 bzw. dem distalen Ende 112 des Katheterschafts 110. Der Führungsdraht verfügt zudem mit Vorteil über eine ausreichende Länge, so dass der erste Katheter 100 bereits ausserhalb des menschlichen oder tierischen Körpers vollständig auf dem Führungsdraht aufgeschoben werden kann. Damit kann der Führungsdraht bei der Bewegung des ersten Katheters 100 gehalten werden.

Ist der erste Katheter 100 im Hohlorgan positioniert, wird der Führungsdraht vollständig zurückgezogen und gegebenenfalls entfernt.

Nun wird das drahtförmige Element, bzw. der Sondendraht, so weit nach vorne geschoben, dass er aus der vorderen Öffnung bzw. aus der Katheterspitze 113 austritt.

Alsdann kann der erste Katheter 100 entlang dem drahtförmigen Element zurück gezogen werden. Da nur der relativ kurze Abschnitt zwischen der Seitenöffnung 130 und dem distalem Ende 112 auf dem drahtförmigen Element bzw. dem Sondendraht aufgefädelt ist, kann der Katheter 100 problemlos vollständig aus dem Hohlorgan heraus gezogen werden. Im Hohlorgan, z. B. in einer Blutbahn, verbleibt einzig das drahtförmige Element bzw. der Sondendraht.

Die vorstehenden Ausführungsbeispiele und Verwendungen sollen lediglich als illustartive Beispiele dienen, welche im Rahmen der Erfindung beliebig abgewandelt werden können.

So ist es z. B. möglich, bei den beiden Kathetern 100, 200 zusätzliche Seitenöffnungen vorzusehen, welche z. B. zum Einleiten und/oder Absaugen von Fluiden dienen. Ebenso ist es möglich, an den Kathetern zusätzliche Elemente vorzusehen. In Frage kommen z. B. spezielle Katheterspitzen, beaufschlagbare Ballone, röntgendichte Markierungen und/oder weitere Lumen im Katheterschaft. Röntgendichte Markierungen können an der Katheterspitze, z. B. in einem Bereich hinter den hohlzylindrischen Rohrstücken 151, 251 in den Katheterschaft 110, 210 eingebracht werden.

Des Weiteren kann, falls erwünscht, auf die Katheterspitzen 113, 213 verzichtet werden, so dass dem Innendurchmesser der distalen Bereiche 113, 223 entsprechende Öffnungen an den distalen Enden 112, 212 der beiden Katheter 100, 200 vorliegen. Auch möglich ist es, anstelle einer stufenartig verjüngten Katheterspitze eine sich konisch zum distalen Ende verjüngende Katheterspitze vorzusehen.

Es ist auch möglich, die hohlzylindrischen Rohrstücke 151, 251 wegzulassen.

Zusätzlich oder anstelle der gefärbten Materialen der hohlzylindrischen Rohrstücke 151. 251 können auch anderen Markierungen, z.B. direkt am Katheterschaft 110, 210 im Bereich des distalen Endes angebracht werden.

Beim zweiten Katheter 200 können die beiden Seitenöffnungen 230a, 230b auch nicht diametral gegenüberliegend, z. B. auf der gleichen Seite am Katheterschaft, vorliegen. Auch können die Abstände zwischen den beiden Seitenöffnungen in weiteren Bereichen variieren. Ebenso ist es möglich, wenigstens eine zusätzliche Seitenöffnung zum Einführen von drahtförmigen Elementen vorzusehen, welche ebenfalls über eine Einführvorrichtung bzw. eine Einführrinne verfügt.

Es liegt auch im Rahmen der Erfindung, die beiden Einführvorrichtungen 240a, 240b unterschiedlich und z. B. auf eine bestimmte Art von drahtfrömigen Elementen abzustimmen.

Die Seitenöffnungen 230a, 230b des zweiten Katheters 200 können auch unterschiedlich dimensioniert vorliegen, so dass z. B. ein spezifisches drahtförmiges Element nur an einer der beiden Seitenöffnungen 230a, 230b durchgeführt werden kann.

Der Stützdraht 990 des dritten Katheters 900 kann grundsätzlich auch einen ovalen, elliptischen oder nicht runden Querschnitt aufweisen. Der konusförmige verjüngte Abschnitt 993 kann auch länger oder kürzer sein. Ebenso ist es möglich, anstelle oder zusätzlich zum konusförmig verjüngten Abschnitts 993 einen stufenartig sich verjüngenden Abschnitt am Stützdraht 990 vorzusehen.

Des Weiteren kann auch der zweite Katheter 200 aus Fig. 3 mit einem zweiteiligen Schaft wie beim dritten Katheter 900 ausgestattet werden. Entsprechend kann in diesem Fall auch beim zweiten Katheter 200 ein Stützdraht angeordnet werden. Dieser ragt dabei mit Vorteil maximal zum proximalen Abschnitt der zweiten Seitenöffnung 230b.

Die beschriebenen Verfahren lassen sich zudem beliebig an spezifische Gegebenheiten anpassen. So können einzelne Verfahrenschritte weggelassen, durch andere Verfahrensschritte ersetzt und/oder die Reihenfolgen der einzelnen Verfahrensschritte geändert werden, falls dies zweckmässig ist.

Zusammenfassend ist festzustellen, dass ein neuartiger Katheter bereitgestellt wird, welcher flexibler einsetzbar ist und die Durchführung von rationelleren und einfacheren Verfahrensabläufen beim Einbringen von drahtförmigen Elementen in menschliche und/oder tierische Hohlorgane ermöglicht.

## Patentansprüche

1. Einlumen-Katheter (200) zur Einführung in ein menschliches und/oder tierisches Hohlorgan, umfassend einen Katheterschaft (210) mit einem Lumen (220), welches in eine vordere Öffnung an einem distalen Ende (212) des Katheterschafts (210) mündet, wobei der Katheterschaft (210) wenigstens eine vom distalen Ende (212) beabstandete und in das Lumen (220) mündende erste Seitenöffnung (230a) aufweist, wobei an einer Aussenseite (214) des Katheterschafts (210) in einem Bereich der ersten Seitenöffnung (230a) wenigstens eine erste Einführvorrichtung (240a) für ein von aussen in die erste Seitenöffnung (230a) einzuführendes drahtförmiges Element (300, 400, 500) vorliegt, **dadurch gekennzeichnet, dass** eine in das Lumen (220) mündende zweite Seitenöffnung (230b) vorgesehen ist, wobei die zweite Seitenöffnung (230b) in einer proximalen Richtung von der ersten Seitenöffnung (230a) beabstandet ist, derart dass ein erstes drahtförmiges Element (300, 400, 500) gegen ein zweites drahtförmiges Element (300, 400, 500) derart ausgetauscht werden kann, dass beide drahtförmigen Elemente gleichzeitig im Katheter vorliegen.

2. Katheter nach Anspruch 1, **dadurch gekennzeichnet, dass** die zweite Seitenöffnung (230b) diametral gegenüberliegend zur ersten Seitenöffnung (230a) angeordnet ist und insbesondere an der Aussenseite (212) des Katheterschafts (210) eine zweite Einführvorrichtung (240b) für das von aussen in die zweite Seitenöffnung (230b) einzuführende zweite drahtförmiges Element (300, 400, 500) vorliegt.

3. Katheter nach einem der Ansprüche 1 - 2, **dadurch gekennzeichnet, dass** die erste Einführvorrichtung (240a) in einem dem proximalen Ende zugewandten und an die erste Seitenöffnung (230a) angrenzenden Bereich der Aussenseite (214) des Katheterschafts (210) vorliegt.

4. Katheter nach einem der Ansprüche 1 - 3, **dadurch gekennzeichnet, dass** die zweite Einführvorrichtung (240b) der zweiten Seitenöffnung (230b) im Wesentlichen baugleich ausgebildet ist wie die erste Einführvorrichtung (240a) der ersten Seltenöffnung (230a).

5. Katheter nach einem der Ansprüche 1 - 4, **dadurch gekennzeichnet, dass** die erste Einführvorrichtung (240a) und/oder die zweite Einführungsvorrichtung (240b) als sich zur ersten Seitenöffnung (230a) bzw. zur zweiten Seitenöffnung (230b) hin erstreckende Führungsrinnen ausgebildet sind, und wobei insbesondere die Führungsrinnen parallel zu einer Längsmittelachse des Lumens (220) verlaufen und besonders bevorzugt eine Breite der Führungsrinnen im Wesentlichen einem Durchmesser der ersten Seitenöffnung (230a) bzw. der zweiten Seitenöffnung (230b) entspricht.

6. Katheter nach Anspruch 5, **dadurch gekennzeichnet, dass** die Führungsrinne als ein konkav gewölbter Bereich der Aussenseite (214) des Katheterschafts (210) ausgebildet ist, wobei der konkav gewölbte Bereich bevorzugt in Richtung zum distalen Ende (212) eine kontinuierlich zunehmende Tiefe aufweist.

7. Katheter nach einem der Ansprüche 1 - 6, **dadurch gekennzeichnet, dass** die erste Seitenöffnung (230a) und/oder die zweiten Seitenöffnung (230b) zum proximalen Ende (211) hin ausgerichtet sind.

8. Katheter nach einem der Ansprüche 1 - 7, **dadurch gekennzeichnet, dass** in einem Bereich der ersten Seitenöffnung (230a) im Lumen (220) ein seitlicher und in die erste Seitenöffnung (230a) mündender erster kanalartiger Bereich (231a) ausgebildet ist, so dass insbesondere neben dem ersten kanalartigen Bereich (231a) ein erster Durchgangsbereich (222a) im Lumen (220) frei bleibt, wobei insbesondere ein dem distalen Ende (212) zugewandter Abschnitt des ersten kanalartigen Bereichs (231a) im Wesentlichen parallel zur Längsachse des Lumens (220) verläuft.

9. Katheter nach einem der Ansprüche 1 - 8, **dadurch gekennzeichnet, dass** ein Innendurchmesser (223.1) des Lumens (220) in einem Bereich (223) zwischen dem distalen Ende (212) und der ersten Seitenöffnung (230a) grösser ausgebildet ist als in den übrigen Bereichen des Lumens (220).

10. Katheter nach einem der Ansprüche 1 - 9, **dadurch gekennzeichnet, dass** am distalen Ende (212) eine verjüngte Katheterspitze vorliegt (213), wobei die Katheterspitze insbesondere durch einen stufenartig verjüngten Endabschnitt des Katheterschafts und ein stirnseitig und koaxial daran befestigtes hohlzylindrisches Rohrstück gebildet wird, und wobei das hohlzylindrische Rohrstück im Besonderen zweiteilig ausgebildet ist.

11. Katheter nach einem der Ansprüche 1 - 10, **dadurch gekennzeichnet, dass** ein durch die zweite Seitenöffnung (230b) in das Lumen (220) geführter Hilfsdraht (300) vorliegt, welcher bis in den Bereich (223) zwischen der ersten Seitenöffnung (230a) und dem distalen Ende (212) hinein ragt, so dass insbesondere der neben dem ersten kanalartigen Bereich (231a) liegende erste Durchgangsbereich (222a) des Lumens (220) blockiert wird, und dass der Hilfsdraht (300) eine ausserhalb der zweiten Seitenöffnung (230b) vorliegende Verdickung (303) aufweist, welche als Anschlag dient und ein vollständiges Durchführen des Hilfsdrahts (300) durch die zweite Seitenöffnung (230b) verhindert.

12. Katheter nach einem der Ansprüche 1 - 11, **dadurch gekennzeichnet, dass** in einem Bereich der ersten Seitenöffnung (230a) und/oder einem Bereich der zweiten Seitenöffnung (230b) eine Markierung (250a, 250b) am Katheterschaft (210) vorliegt, wobei die Markierung (250a, 250b) insbesondere als Farbmarkierung ausgebildet ist.

13. Katheter nach einem der Ansprüche 1 - 12, **dadurch gekennzeichnet, dass** ein proximaler Abschnitt (910b) des Katheterschafts (910) eine geringere Elastizität aufweist als ein distaler Abschnitt (910a) des Katheterschafts (910), wobei insbesondere der proximale Abschnitt (910b) des Katheterschafts ein Metall röhrchen umfasst und der distale Abschnitt (910a) des Katheterschafts ein Kunststoffröhrchen beinhaltet,

14. Katheter nach Anspruch 13, **dadurch gekennzeichnet, dass** in einem Übergangsbereich zwischen dem proximalen Abschnitt (910b) und dem distalen Abschnitt (910a) des Katheterschafts (910) ein Stützelement (990) angeordnet ist, insbesondere ein in longitudinaler Richtung verlaufender Stützdraht, wobei das Stützelement (990) so ausgebildet ist, dass ein Elastizitätssprung zwischen dem proximalen Abschnitt (910b) und dem distalen Abschnitt (910a) des Katheterschafts reduziert und/oder wenigstens teilweise ausgeglichen wird.

15. Katheter nach Anspruch 14, **dadurch gekennzeichnet, dass** das Stützelement (990) ein Stützdraht ist, welcher am Metallröhrchen (910b) angebracht ist und in den Innenbereich des distalen Abschnitts des Kunststoffröhrchens (910a) hinein ragt, und dass insbesondere das Stützelement (990) derart am Metallröhrchen (910b) angebracht, dass ein innerer Hohlraum des Metallröhrchens (910b) vollständig frei bleibt.

16. Katheter nach einem der Ansprüche -14 - 15, **dadurch gekennzeichnet, dass** das Metallröhrchen (910b) mit Vorteil über einen in das Metallröhrchen (910b) eingebrachten Schlitz (913b), welcher sich vom distalen Ende (912b) des Metallröhrchens (910b) in longitudinaler Richtung des Metallröhrchens (910b) erstreckt, verfügt, wobei der Schlitz (913b) zur tellweisen Aufnahme des Stützelements (990) ausgebildet ist, und dass sich das Stützelement (990) bevorzugt in distaler Richtung maximal bis zu einem proximalen Bereich der wenigstens einen Seitenöffnung (230b) hin erstreckt.

17. Katheter nach einem der Ansprüche 14 - 16, **dadurch gekennzeichnet, dass** das Kunststoffröhrchen (910a) mit seinem proximalen Ende (911a) auf das distale Ende (912b) des Metallröhrchens (910b) aufgeschoben und am distalen Ende (912b) des Metallröhrchens (910b) befestigt ist, insbesondere durch Pressschweissung.

18. Gerätesatz, umfassend einen Katheter (200) nach einem der Ansprüche 1 - 17, einen Führungsdraht (400) sowie einen Spezialdraht (500) für diagnostische, chirurgische und/oder therapeutische Zwecke.

19. Gerätesatz nach Anspruch 18, **dadurch gekennzeichnet, dass** ein Aussendurchmesser des Führungsdrahts (400) und ein Aussendurchmesser des Spezialdrahts (500) zusammen höchstens ungefähr dem Innendurchmesser des Lumens in Bereich (223) zwischen dem distalen Ende (212) und der ersten Seitenöffnung (230a) entspricht, wobei ein Durchmesser der ersten Seitenöffnung (230a) in etwa dem Aussendurchmesser des Führungsdrahts (400) entspricht, während ein Durchmesser der zweiten Seitenöffnung (230b) in etwa dem Aussendurchmesser das Spezialdrahts (500) entspricht.

20. Gerätesatz nach einem der Ansprüche 18 - 19, **dadurch gekennzeichnet, dass** der Spezialdraht (500) als Sondendraht für ein tomographisches Verfahren ausgebildet ist, wobei es sich insbesondere um einen Sondendraht für eine optische Kohärenztomographie handelt.

## Claims

1. A single-lumen catheter (200) for insertion into a human and/or animal hollow organ, comprising a catheter shaft (210) with a lumen (220), which opens into a front opening at a distal end (212) of the catheter shaft (210), wherein the catheter shaft (210) has at least a first lateral opening (230a) that is at a distance from the distal end (212) and opens into the lumen (220), wherein there is at least a first insertion device (240a), arranged on an external side (214) of the catheter shaft (210) in a region of the first lateral opening (230a), for a wire-like element (300, 400, 500) that should be inserted into the first lateral opening (230a) from the outside, **characterized in that** there is a second lateral opening (230b) that opens into the lumen (220), wherein the second lateral opening (230b) is spaced apart from the first lateral opening (230a) in a proximal direction, such that a first wire-like element (300, 400, 500) can be exchanged for a second wire-like element (300, 400, 500), such that both wire-like elements are simultaneously present in the catheter.

2. The catheter as claimed in claim 1, **characterized in that** the second lateral opening (230b) is arranged diametrically opposite to the first lateral opening (230a) and there is, particularly on the external side (212) of the catheter shaft (210), a second insertion device (240b) for a second wire-like element (300, 400, 500) that should be inserted into the second lateral opening (230b) from the outside.

3. The catheter as claimed in one of claims 1 - 2, **characterized in that** the first insertion device (240a) is in a region of the external side (214) of the catheter shaft (210) that faces the proximal end and adjoins the first lateral opening (230a).

4. The catheter as claimed in one of claims 1 - 3, **characterized in that** the second insertion device (240b) for the second lateral opening (230b) substantially has the same design as the first insertion device (240a) for the first lateral opening (230a).

5. The catheter as claimed in one of claims 1 - 4, **characterized in that** the first insertion device (240a) and/or the second insertion device (240b) is embodied as a guide trough that extends to the first lateral opening (230a) or to the second lateral opening (230b), respectively, and wherein, more particularly, the guide trough runs parallel to a longitudinal central axis of the lumen (220) and, particularly preferably, a width of the guide trough substantially corresponds to a diameter of the first lateral opening (230a) or of the second lateral opening (230b), respectively.

6. The catheter as claimed in claim 5, **characterized in that** the guide trough is embodied as a concavely arced region of the external side (214) of the catheter shaft (210), wherein the concavely arced region preferably has a continuously increasing depth in the direction toward the distal end (212).

7. The catheter as claimed in one of claims 1 - 6, **characterized in that** the first lateral opening (230a) and/or the second lateral opening (230b) are aligned with the proximal end (211).

8. The catheter as claimed in one of claims 1 - 7, **characterized in that** a lateral first channel-like region (231a), which opens into the first lateral opening (230a), is formed in the lumen (220) in a region of the first lateral opening (230a) such that a first passage region (222a) in the lumen (220) remains free, whereby, particularly, a section of the first channel-like region (231a) facing the distal end (212) runs substantially parallel to the longitudinal axis of the lumen (220).

9. The catheter as claimed in one of claims 1 - 8, **characterized in that** an internal diameter (223.1) of the lumen (220) is larger in a region (223) between the distal end (212) and the first lateral opening (230a) than in the remaining regions of the lumen (220).

10. The catheter as claimed in one of claims 1 - 9 **characterized in that** there is (213) a tapered catheter tip at the distal end (212), wherein the catheter tip is more particularly formed by a step-like tapered end section of the catheter shaft and a hollow cylindrical tube piece, which is attached thereto in a coaxial fashion and on the end face, and wherein, more particularly, the hollow cylindrical tube piece has a two-part design.

11. The catheter as claimed in one of claims 1 - 10, **characterized in that** there is an auxiliary wire (300), which is routed through the second lateral opening (230b) into the lumen (220) and which projects into the region (223) between the first lateral opening (230a) and distal end (212), and so, more particularly, the first passage region (222a) of the lumen (220), situated next to the first channel-like region (231a), is blocked, and the auxiliary wire (300) has a thickening (303) situated outside of the second lateral opening (230b), which thickening serves as a stop and prevents the auxiliary wire (300) from completely passing through the second lateral opening (230b).

12. The catheter as claimed in one of claims 1 - 11, **characterized in that** there is a marking (250a, 250b) on the catheter shaft (210) in a region of the first lateral opening (230a) and/or in a region of the second lateral opening (230b), wherein the marking (250a, 250b) is more particularly embodied as a colored marking.

13. The catheter as claimed in one of claims 1 - 12, **characterized in that** a proximal section (910b) of the catheter shaft (910) has a lower elasticity than a distal section (910a) of the catheter shaft (910), wherein, particularly, the proximal section (910b) of the catheter shaft comprises a metal tubule and the distal section (910a) of the catheter shaft contains a plastic tubule.

14. The catheter as claimed in claim 13, **characterized in that** a support element (990), more particularly a support wire running in the longitudinal direction, is arranged in a transition region between the proximal section (910b) and the distal section (910a) of the catheter shaft (910), wherein the support element (990) is embodied such that a jump in elasticity between the proximal section (910b) and the distal section (910a) of the catheter shaft is reduced and/or at least partly compensated for.

15. The catheter as claimed in claim 14, **characterized in that** the support element (990) is a support wire, which is attached to the metal tubule (910b) and projects into the internal region of the distal section of the plastic tubule (910a), and, particularly, the support element (990) is attached to the metal tubule (910b) such that an inner cavity of the metal tubule (910b) remains completely free.

16. The catheter as claimed in one of claims 14 - 15, **characterized in that** the metal tubule (910b) advantageously comprises a slit (913b) introduced into the metal tubule (910b), which slit extends from the distal end (912b) of the metal tubule (910b) in the longitudinal direction of the metal tubule (910b), wherein the slit (913b) is embodied for partly holding the support element (990), and, the support element (990) preferably at most extends in the distal direction to a proximal region of the at least one lateral opening (230b).

17. The catheter as claimed in one of claims 14 - 16, **characterized in that** the proximal end (911 a) of the plastic tubule (910a) is pushed onto the distal end (912b) of the metal tubule (910b) and attached to the distal end (912b) of the metal tubule (910b), more particularly by pressure welding.

18. A set of instruments, comprising a catheter (200) as claimed in one of claims 1 - 17, a guide wire (400), and a special wire (500) for diagnostic, surgical, and/or therapeutic purposes.

19. The set of instruments as claimed in claim 18, **characterized in that** an external diameter of the guide wire (400) and an external diameter of the special wire (500) together at most correspond approximately to the internal diameter of the lumen in the region (223) between the distal end (212) and the first lateral opening (230a), wherein a diameter of the first lateral opening (230a) approximately corresponds to the external diameter of the guide wire (400), while the second lateral opening (230b) has a diameter that approximately corresponds to the external diameter of the special wire (500).

20. The set of instruments as claimed in one of claims 18 - 19, **characterized in that** the special wire (500) is embodied as a probe wire for a tomographic method, wherein, more particularly, this is a probe wire for optical coherence tomography.

## Revendications

1. Cathéter à simple lumière (200) destiné à être inséré dans un organe creux humain et/ou animal, comportant une tige de cathéter (210) munie d'une lumière (220), qui débouche dans une ouverture avant à une extrémité distale (212) de la tige de cathéter (210), la tige de cathéter (210) comprenant au moins une première ouverture latérale (230a), espacée de l'extrémité distale (212) et débouchant dans la lumière (220), au moins un premier dispositif d'insertion (240a) pour un élément en forme de fil (300, 400, 500), à insérer depuis l'extérieur dans la première ouverture latérale (230a) étant présent sur un côté extérieur (214) de la tige de cathéter (210) dans une zone de la première ouverture latérale (230a), **caractérisé en ce qu'**une deuxième ouverture latérale (230b) débouchant dans la lumière (220) est prévue, la deuxième ouverture latérale (230b) étant espacée de la première ouverture latérale (230a) dans une direction proximale, de telle sorte qu'un premier élément en forme de fil (300, 400, 500) puisse être remplacé par un deuxième élément en forme de fil (300, 400, 500), de telle sorte que les deux éléments en forme de fil soient présents simultanément dans le cathéter.

2. Cathéter selon la revendication 1, **caractérisé en ce que** la deuxième ouverture latérale (230b) est agencée de manière diamétralement opposée à la première ouverture latérale (230a) et notamment un deuxième dispositif d'insertion (240b) pour le deuxième élément en forme de fil (300, 400, 500) à insérer depuis l'extérieur dans la deuxième ouverture latérale (230b) est présent sur le côté extérieur (212) de la tige de cathéter (210).

3. Cathéter selon l'une quelconque des revendications 1 à 2, **caractérisé en ce que** le premier dispositif d'insertion (240a) est présent dans une zone, tournée vers l'extrémité proximale et adjacente à la première ouverture latérale (230a), du côté extérieur (214) de la tige de cathéter (210).

4. Cathéter selon l'une quelconque des revendications 1 à 3, **caractérisé en ce que** le deuxième dispositif d'insertion (240b) de la deuxième ouverture latérale (230b) est configuré de manière essentiellement identique au premier dispositif d'insertion (240a) de la première ouverture latérale (230a).

5. Cathéter selon l'une quelconque des revendications 1 à 4, **caractérisé en ce que** le premier dispositif d'insertion (240a) et/ou le deuxième dispositif d'insertion (240b) sont configurés sous la forme de conduits de guidage s'étendant jusqu'à la première ouverture latérale (230a) ou jusqu'à la deuxième ouverture latérale (230b), et dans lequel, notamment, les conduits de guidage sont parallèles à un axe central longitudinal de la lumière (220) et, de manière particulièrement préférée, une largeur des conduits de guidage correspond essentiellement à un diamètre de la première ouverture latérale (230a) ou de la deuxième ouverture latérale (230b).

6. Cathéter selon la revendication 5, **caractérisé en ce que** le conduit de guidage est configuré sous la forme d'une zone à courbure concave du côté extérieur (214) de la tige de cathéter (210), la zone à courbure concave présentant de préférence une profondeur continuellement croissante en direction de l'extrémité distale (212).

7. Cathéter selon l'une quelconque des revendications 1 à 6, **caractérisé en ce que** la première ouverture latérale (230a) et/ou la deuxième ouverture latérale (230b) sont orientées vers l'extrémité proximale (211).

8. Cathéter selon l'une quelconque des revendications 1 à 7, **caractérisé en ce qu'**une première zone de type canal (231a) latérale et débouchant dans la première ouverture latérale (230a) est formée dans une zone de la première ouverture latérale (230a) dans la lumière (220), de telle sorte qu'une première zone de passage (222a) reste notamment libre dans la lumière (220) à côté de la première zone de type canal (231a), une section, tournée vers l'extrémité distale (212), de la première zone de type canal (231a) étant notamment essentiellement parallèle à l'axe longitudinal de la lumière (220).

9. Cathéter selon l'une quelconque des revendications 1 à 8, **caractérisé en ce qu'**un diamètre intérieur (223.1) de la lumière (220) dans une zone (223) entre l'extrémité distale (212) et la première ouverture latérale (230a) est configuré plus grand que dans les autres zones de la lumière (220).

10. Cathéter selon l'une quelconque des revendications 1 à 9, **caractérisé en ce qu'**une pointe de cathéter effilée (213) est présente à l'extrémité distale (212), la pointe de cathéter étant notamment formée par une section d'extrémité effilée de manière étagée de la tige de cathéter et un tronçon de tuyau cylindrique creux fixé du côté frontal et coaxialement à celle-ci, et le tronçon de tuyau cylindrique creux étant notamment configuré en deux parties.

11. Cathéter selon l'une quelconque des revendications 1 à 10, **caractérisé en ce qu'**un fil auxiliaire (300) guidé dans la lumière (220) au travers de la deuxième ouverture latérale (230b) est présent, qui s'enfonce jusque dans la zone (223) entre la première ouverture latérale (230a) et l'extrémité distale (212), de telle sorte que la première zone de passage (222a), située à côté de la première zone de type canal (231a), de la lumière (220) soit bloquée, et **en ce que** le fil auxiliaire (300) présente un épaississement (303) présent à l'extérieur de la deuxième ouverture latérale (230b), qui sert de butée et empêche un passage complet du fil auxiliaire (300) au travers de la deuxième ouverture latérale (230b).

12. Cathéter selon l'une quelconque des revendications 1 à 11, **caractérisé en ce qu'**un marquage (250a, 250b) est présent sur la tige de cathéter (210) dans une zone de la première ouverture latérale (230a) et/ou une zone de la deuxième ouverture latérale (230b), le marquage (250a, 250b) étant notamment configuré sous la forme d'un marquage de couleur.

13. Cathéter selon l'une quelconque des revendications 1 à 12, **caractérisé en ce qu'**une section proximale (910b) de la tige de cathéter (910) présente une élasticité plus faible qu'une section distale (910a) de la tige de cathéter (910), la section proximale (910b) de la tige de cathéter comprenant notamment un tube métallique et la section distale (910a) de la tige de cathéter contenant un tube en matière plastique.

14. Cathéter selon la revendication 13, **caractérisé en ce qu'**un élément support (990) est agencé dans une zone de transition entre la section proximale (910b) et la section distale (910a) de la tige de cathéter (910), notamment un fil support s'étendant en direction longitudinale, l'élément support (990) étant configuré de telle sorte qu'une discontinuité d'élasticité entre la section proximale (910b) et la section distale (910a) de la tige de cathéter soit réduite et/ou au moins partiellement équilibrée.

15. Cathéter selon la revendication 14, **caractérisé en ce que** l'élément support (990) est un fil support, qui est disposé sur le tube métallique (910b) et s'enfonce dans la zone intérieure de la section distale du tube en matière plastique (910a), et **en ce que** l'élément support (990) est notamment disposé sur le tube métallique (910b) de telle sorte qu'une cavité intérieure du tube métallique (910b) reste entièrement libre.

16. Cathéter selon l'une quelconque des revendications 14 à 15, **caractérisé en ce que** le tube métallique (910b) dispose avantageusement d'une fente (913b) aménagée dans le tube métallique (910b), qui s'étend à partir de l'extrémité distale (912b) du tube métallique (910b) en direction longitudinale du tube métallique (910b), la fente (913b) étant configurée pour la réception partielle de l'élément support (990), et **en ce que** l'élément support (990) s'étend de préférence en direction distale au plus jusqu'à une zone proximale de ladite au moins une ouverture latérale (230b).

17. Cathéter selon l'une quelconque des revendications 14 à 16, **caractérisé en ce que** le tube en matière plastique (910a) est enfilé avec son extrémité proximale (911a) sur l'extrémité distale (912b) du tube métallique (910b) et est fixé à l'extrémité distale (912b) du tube métallique (910b), notamment par soudage avec pression.

18. Ensemble, comprenant un cathéter (200) selon l'une quelconque des revendications 1 à 17, un fil de guidage (400), ainsi qu'un fil spécial (500) à des fins diagnostiques, chirurgicales et/ou thérapeutiques.

19. Ensemble selon la revendication 18, **caractérisé en ce qu'**un diamètre extérieur du fil de guidage (400) et un diamètre extérieur du fil spécial (500) correspondent ensemble au plus approximativement au diamètre intérieur de la lumière dans la zone (223) entre l'extrémité distale (212) et la première ouverture latérale (230a), un diamètre de la première ouverture latérale (230a) correspondant environ au diamètre extérieur du fil de guidage (400), tandis qu'un diamètre de la deuxième ouverture latérale (230b) correspond environ au diamètre extérieur du fil spécial (500) .

20. Ensemble selon l'une quelconque des revendications 18 à 19, **caractérisé en ce que** le fil spécial (500) est configuré sous la forme d'un fil de sonde pour un procédé tomographique, celui-ci consistant notamment en un fil de sonde pour une tomographie en cohérence optique.
